# EUROPÄISCHE PATENTSCHRIFT

(11) **EP 2 276 723 B1**
(45) Veröffentlichungstag und Bekanntmachung des Hinweises auf die Patenterteilung: **27.01.2016**
(21) Anmeldenummer: 09749516.2
(22) Anmeldetag: 05.03.2009
(51) Int. Cl.: C07C 211/61, C09K 11/06, H01L 51/00, H01L 51/50, H05B 33/22

(54) **VERBINDUNGEN FÜR ELEKTRONISCHE VORRICHTUNGEN**
COMPOUNDS FOR ELECTRONIC DEVICES
COMPOSÉS POUR DISPOSITIFS ÉLECTRONIQUES

(30) Priorität: 19.05.2008 DE 102008024182
(43) Veröffentlichungstag der Anmeldung: 26.01.2011
(73) Patentinhaber: Merck Patent GmbH, 64293 Darmstadt (DE)
(72) Erfinder: PFLUMM, Christof, 60316 Frankfurt (DE); BUESING, Arne, 65929 Frankfurt am Main (DE); PARHAM, Amir, Hossain, 65929 Frankfurt (DE); FORTTE, Rocco, 65933 Frankfurt (DE); HEIL, Holger, 60389 Frankfurt (DE); STOESSEL, Philipp, 60487 Frankfurt am Main (DE)
(86) Internationale Anmeldenummer: PCT/EP2009/001554
(87) Internationale Veröffentlichungsnummer: WO 2009/141026

(56) Entgegenhaltungen:
- EP-A- 1 860 097
- WO-A-2006/122630

## Beschreibung

Die vorliegende Erfindung beschreibt neue Verbindungen und deren Einsatz in elektronischen Vorrichtungen.

Der allgemeine Aufbau organischer Elektrolumineszenzvorrichtungen ist beispielsweise in US 4539507, US 5151629, EP 0676461 und WO 98/27136 beschrieben. Allerdings zeigen diese Vorrichtungen immer noch erhebliche Probleme, die einer dringenden Verbesserung bedürfen:
1. Die Effizienz ist gerade bei fluoreszierenden OLEDs immer noch niedrig und sollte verbessert werden.
2. Bei der operativen Lebensdauer besteht insbesondere bei blauer Emission immer noch Verbesserungsbedarf.
3. Die Betriebsspannung ist gerade bei fluoreszierenden OLEDs recht hoch und sollte daher weiter verringert werden, um die Leistungseffizienz zu verbessern. Das ist insbesondere für mobile Anwendungen von großer Bedeutung. Hier sind weitere Verbesserungen insbesondere bei Ladungstransportmaterialien wünschenswert.
4. Bei Lochtransportmaterialien gemäß dem Stand der Technik ist die Spannung abhängig von der Schichtdicke der Lochtransportschicht. In der Praxis wäre häufig eine dickere Schichtdicke der Lochtransportschicht wünschenswert. Dies lässt sich jedoch wegen des damit verbundenen Spannungsanstiegs mit Materialien gemäß dem Stand der Technik kaum realisieren.
5. Bei Lochtransportmaterialien gemäß dem Stand der Technik gibt es häufig Probleme mit der Prozessierbarkeit, da diese Materialien am Rand der Aufdampfquelle auskristallisieren und so die Aufdampfquelle verstopfen ("clogging"). Derartige Materialien können daher nur unter erhöhtem technischen Aufwand in der Massenproduktion eingesetzt werden.

Der nächstliegender Stand der Technik für Lochinjektions- und -transportmaterialien sind cis- und trans-Indenofluoren-Derivate, welche mit einer oder zwei Diarylaminogruppen substituiert sind, gemäß WO 06/100896 und WO 06/122630. Zwar sind darin generell auch substituierte Indenofluoren-Derivate umfasst, jedoch sind nur Indenofluorene mit unsubstituiertem Indenofluoren-Grundkörper explizit offenbart. Es sind keine Beispiele mit substituierten Indenofluorenstrukturen offenbart, und insbesondere ist nicht offenbart, an welchen Positionen Substituenten zu besonders guten Ergebnissen führen und welche Substituenten zu Verbesserungen führen. Es ist lediglich offenbart, dass gerade die unsubstituierten Indenofluorene besonders bevorzugt sind. Es hat sich jedoch gezeigt, dass diese bei Verwendung in einer Lochinjektions- bzw. Lochtransportschicht zu hohen Betriebsspannungen führen, so dass hier noch weitere Verbesserungen erforderlich sind. Weiterhin gibt es mit derartigen Materialien häufig Probleme, da diese durch Kristallisation die Aufdampfquelle verstopfen.

Es wurde nun überraschend gefunden, dass Indenofluoren-Derivate mit einem oder zwei Diarylamino-Substituenten, welche am Indenofluoren-Grundkörper in bestimmten Positionen substituiert sind, hier deutliche Verbesserungen aufweisen. Mit diesen Verbindungen ist eine Reduktion der Betriebsspannung bei gleichzeitig unverändert guter Effizienz und Lebensdauer möglich im Vergleich zu den entsprechenden unsubstituierten Verbindungen. Diese Verbindungen und deren Verwendung in OLEDs sind daher der Gegenstand der vorliegenden Erfindung.

Gegenstand der Erfindung sind daher Verbindungen gemäß Formel (1), 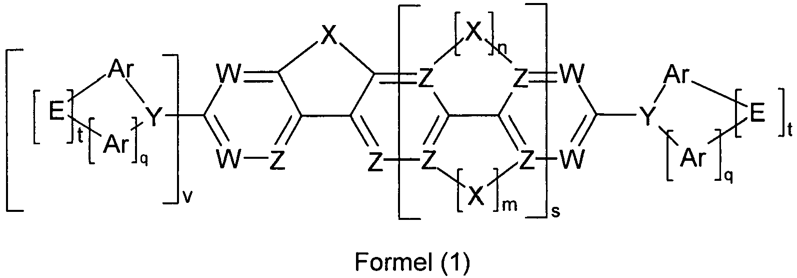 wobei für die verwendeten Symbole und Indizes gilt:
- Y: ist N;
- Ar: ist bei jedem Auftreten gleich oder verschieden ein aromatisches oder heteroaromatisches Ringsystem mit 5 bis 40 aromatischen Ringatomen, das mit einem oder mehreren Resten R¹ substituiert sein kann;
- X: ist bei jedem Auftreten gleich oder verschieden eine Gruppe, ausgewählt aus C(R¹)₂ und Si(R¹)₂;
- Z: ist C, wenn an die Gruppe Z eine Gruppe X gebunden ist, oder ist CR, wenn an die Gruppe Z keine Gruppe X gebunden ist;
- W: ist CH;
- R: ist bei jedem Auftreten gleich oder verschieden H, D, eine geradkettige Alkylgruppe mit 1 bis 10 C-Atomen oder eine verzweigte oder cyclische Alkylgruppe mit 3 bis 10 C-Atomen, die jeweils mit einem oder mehreren Resten R² substituiert sein kann;
- E: ist bei jedem Auftreten gleich oder verschieden eine Einfachbindung, N(R¹), O, S, C(R¹)₂, C(R¹)₂-C(R¹)₂, Si(R¹)₂ oder B(R¹);
- R¹: ist bei jedem Auftreten gleich oder verschieden H, D, F, Cl, Br, I, CN, NO₂ B(OR²)₂, Si(R²)₃, eine geradkettige Alkyl-, Alkoxy- oder Thio-alkoxygruppe mit 1 bis 40 C-Atomen oder eine geradkettige Alkenyl-oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch -R²C=CR²-, -C=C-, Si(R²)₂, Ge(R²)₂, Sn(R²)₂, C=O, C=S, C=Se, C=NR², -O-, -S-, -COO- oder -CONR²- ersetzt sein können und wobei ein oder mehrere H-Atome durch F, Cl, Br, I, CN oder NO₂ ersetzt sein können, oder ein aromatisches oder hetero-aromatisches Ringsystem mit 5 bis 40 aromatischen Ringatomen, das durch einen oder mehrere nicht-aromatische Reste R¹ substituiert sein kann, oder eine Aryloxy- oder Heteroaryloxygruppe mit 5 bis 40 aromatischen Ringatomen, die durch einen oder mehrere nicht-aromatische Reste R¹ substituiert sein kann, oder eine Kombination dieser Systeme; dabei können zwei oder mehrere Substituenten R¹ auch miteinander ein mono- oder polycyclisches Ring-system bilden;
- R²: ist bei jedem Auftreten gleich oder verschieden H oder ein aliphatischer oder aromatischer Kohlenwasserstoffrest mit 1 bis 20 C-Atomen;
- m, n: sind 0 oder 1, mit der Maßgabe, dass m + n = 1 ist;
- q: ist bei jedem Auftreten 1;
- s: ist 1 oder 2;
- t: ist bei jedem Auftreten gleich oder verschieden 0 oder 1, wobei t = 0 bedeutet, dass statt der Gruppe E Reste R¹ gebunden sind; dabei gilt weiterhin, dass t = 0 ist, wenn q = 0 ist;
- v: ist 0 oder 1, wobei für v = 0 statt der Gruppe Y Wasserstoff oder eine Gruppe R gebunden sein kann;
dadurch gekennzeichnet, dass mindestens ein Rest R einen Substituenten ungleich H oder D darstellt.

Unter benachbarten Substituenten im Sinne dieser Erfindung werden Substituenten verstanden, welche entweder an dasselbe Atom gebunden sind, also beispielsweise die beiden Substituenten R¹ in einer Gruppe C(R¹)₂, oder Substituenten, welche an direkt benachbarte Atome gebunden sind, also beispielsweise die beiden Substituenten R in einer Gruppe C(R)-C(R).

Unter einer Arylgruppe bzw. einer Heteroarylgruppe im Sinne dieser Erfindung wird eine aromatische Gruppe bzw. heteroaromatische Gruppe mit einem gemeinsamen aromatischen Elektronensystem verstanden, wobei eine Arylgruppe 6 bis 24 C-Atome und eine Heteroarylgruppe 2 bis 24 C-Atome und insgesamt mindestens 5 aromatische Ringatome umfasst. Die Heteroatome sind bevorzugt ausgewählt aus N, O und/oder S. Dies kann im Sinne dieser Erfindung ein einfacher Homo- oder Heterocyclus sein, beispielsweise Benzol, Pyridin, Thiophen, etc., oder es kann ein kondensiertes aromatisches Ringsystem sein, in dem mindestens zwei aromatische oder heteroaromatische Ringe, beispielsweise Benzolringe, miteinander "verschmolzen", d. h. durch Anellierung einander ankondensiert sind, also mindestens eine gemeinsame Kante und dadurch auch ein gemeinsames aromatisches System aufweisen. Diese Aryl- oder Heteroarylgruppe kann substituiert oder unsubstituiert sein; ebenso können gegebenenfalls vorhandene Substituenten weitere Ringsysteme bilden. So sind beispielsweise Systeme wie Naphthalin, Anthracen, Phenanthren, Pyren, etc. als Arylgruppen und Chinolin, Acridin, Benzothiophen, Carbazol, etc. als Heteroarylgruppen im Sinne dieser Erfindung zu sehen, während beispielsweise Biphenyl, Fluoren, Spirobifluoren, etc. keine Arylgruppen darstellen, da es sich hierbei um separate aromatische Elektronensysteme handelt.

Ein aromatisches Ringsystem im Sinne dieser Erfindung enthält 6 bis 40 C-Atome im Ringsystem. Ein heteroaromatisches Ringsystem im Sinne dieser Erfindung enthält 2 bis 40 C-Atome und mindestens ein Heteroatom im Ringsystem, mit der Maßgabe, dass die Summe der C-Atome und Heteroatome mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine kurze, nicht-aromatische Einheit (weniger als 10 % der von H verschiedenen Atome, bevorzugt weniger als 5 % der von H verschiedenen Atome), wie z. B. ein C-, N- oder O-Atom, unterbrochen sein können. So sollen beispielsweise auch Systeme wie 9,9'-Spirobifluoren, 9,9-Diarylfluoren, Triarylamin, Diarylether, etc. als aromatische Ringsysteme im Sinne dieser Erfindung verstanden werden.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, tert-Penyl, 2-Pentyl, Cyclopentyl, n-Hexyl, s-Hexyl, tert-Hexyl, 2-Hexyl, 3-Hexyl, Cyclohexyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer C₁- bis C₄₀-Alkoxygruppe werden besonders bevorzugt Methoxy, Ethoxy, n-Propoxy, i-Propoxy, n-Butoxy, i-Butoxy, s-Butoxy, t-Butoxy oder 2-Methylbutoxy verstanden. Unter einer C₂-C₂₄ Aryl- oder Heteroarylgruppe, die je nach Verwendung monovalent oder bivalent sein kann, die noch jeweils mit den oben genannten Resten R¹ substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol. Unter aromatischen und heteroaromatischen Ringsystemen im Sinne dieser Erfindung werden außer den oben genannten Aryl- und Heteroarylgruppen insbesondere Biphenylen, Terphenylen, Fluoren, Spirobifluoren, Dihydrophenanthren, Tetrahydropyren und cis- oder trans-Indenofluoren verstanden.

Bevorzugt sind weiterhin Verbindungen mit s = 1.

Bevorzugte Ausführungsformen der Erfindung sind die Verbindungen gemäß Formel (2) oder (3), 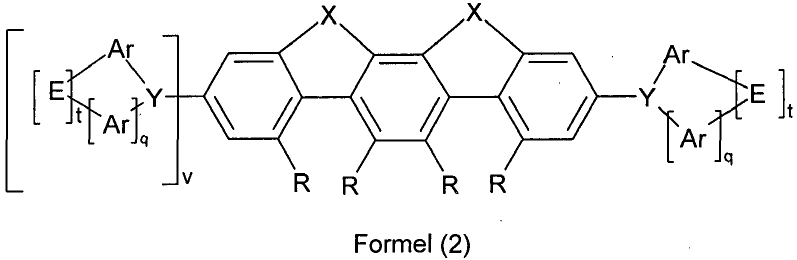 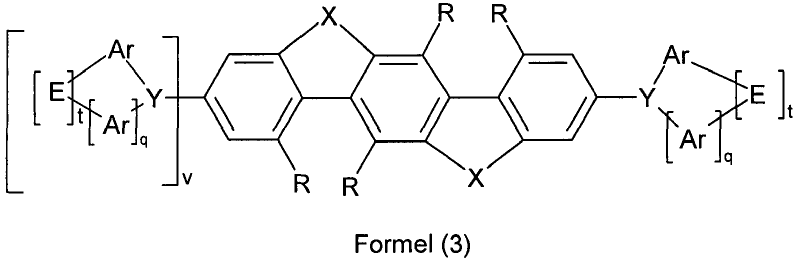 wobei die verwendeten Symbole und Indizes die oben aufgeführten Bedeutungen haben und mindestens eine Gruppe R in Formel (2) und (3) für einen Substituenten ungleich Wasserstoff oder Deuterium steht.

Weiterhin bevorzugt sind Verbindungen gemäß Formel (1) bis (3), in denen der Index v = 1 ist.

Bevorzugt sind weiterhin Verbindungen gemäß Formel (1) bis (3), in denen die Symbole Ar gleich oder verschieden bei jedem Auftreten für ein aromatisches oder heteroaromatisches Ringsystem mit 5 bis 16 aromatischen Ringatomen, für ein Triarylamin oder für Spirobifluoren stehen, das jeweils mit einem oder mehreren Resten R¹ substituiert sein kann, besonders bevorzugt für ein aromatisches oder heteroaromatisches Ringsystem, ausgewählt aus Benzol, ortho-, meta- oder para-Biphenyl, Fluoren, Naphthalin, Anthracen, Phenanthren, Benzanthracen, Pyridin, Pyren, Thiophen, Triphenylamin, Diphenyl-1-naphthylamin, Diphenyl-2-naphthylamin, Phenyl-di(1-naphthyl)amin und Phenyl-di(2-naphthyl)amin, welches jeweils mit R¹ substituiert sein kann. Ganz besonders bevorzugt stehen die Symbole Ar gleich oder verschieden bei jedem Auftreten für Phenyl, 1-Naphthyl oder 2-Naphthyl, das jeweils mit einem oder zwei Resten R¹ substituiert sein kann.

Bevorzugt sind weiterhin Verbindungen gemäß Formel (1) bis (3), in denen der Index t = 0 ist oder in denen der Index t = 1 ist und das entsprechende Symbol E für eine Einfachbindung, C(R¹)₂, S oder N(R¹) steht. Besonders bevorzugt sind Verbindungen gemäß Formel (1) bis (3), in denen der Index t = 0 ist oder in denen der Index t = 1 ist und das entsprechende Symbol E für eine Einfachbindung oder C(R¹)₂ steht.

Bevorzugt sind weiterhin Verbindungen gemäß Formel (1) bis (3), in denen das Symbol R¹ gleich oder verschieden bei jedem Auftreten für H, F, CN, eine geradkettige Alkylgruppe mit 1 bis 5 C-Atomen oder eine verzweigte Alkylgruppe mit 3 bis 5 C-Atomen, wobei jeweils eine oder mehrere nicht benachbarte CH₂-Gruppen durch -R²C=CR²-, -O- oder -S-ersetzt sein können und wobei ein oder mehrere H-Atome durch F ersetzt sein können, oder eine monovalente Aryl- oder Heteroarylgruppe mit 5 bis 16 aromatischen Ringatomen, die mit einem oder mehreren nicht-aromatischen Resten R¹ substituiert sein kann, steht, wobei zwei oder mehrere Reste R¹ miteinander ein Ringsystem bilden können; besonders bevorzugt steht R¹ für H, F, CN, Methyl, tert-Butyl oder eine monovalente Aryl- oder Heteroarylgruppe mit 4 bis 6 C-Atomen, die mit einem oder mehreren nicht-aromatischen Resten R¹ substituiert sein kann, wobei zwei aromatische Reste R¹ miteinander ein Ringsystem bilden können.

Weiterhin bevorzugt ist R¹, wenn es an eine Gruppe X gebunden ist, eine geradkettige Alkylgruppe mit 1 bis 10 C-Atomen oder eine verzweigte oder cyclische Alkylgruppe mit 3 bis 10 C-Atomen, wobei jeweils eine oder mehrere nicht benachbarte CH₂-Gruppen durch -R²C=CR²-, -O- oder -S-ersetzt sein können und wobei ein oder mehrere H-Atome durch F ersetzt sein können, oder eine monovalente Aryl- oder Heteroarylgruppe mit 5 bis 16 aromatischen Ringatomen, die mit einem oder mehreren nicht-aromatischen Resten R¹ substituiert sein kann; dabei können auch zwei benachbarte Reste R¹ miteinander ein Ringsystem bilden. Besonders bevorzugt sind die Reste R¹ ausgesucht aus geradkettigen Alkylgruppen mit 1 bis 4 C-Atomen oder verzweigten Alkylgruppen mit 3 oder 4 C-Atomen, insbesondere Methylgruppen, oder Phenylgruppen; dabei können zwei oder mehrere Reste R¹ miteinander ein Ringsystem bilden. Wenn mehrere Reste R¹ miteinander ein Ringsystem bilden, wird hierdurch eine Spiro-Struktur gebildet. Dies kann insbesondere dann bevorzugt sein, wenn die Reste R¹ für Phenylgruppen stehen.

In den Verbindungen gemäß Formel (2) und (3) ist mindestens ein Substituent R ungleich H oder D. Dabei ist es auch möglich, dass mehrere Substituenten R ungleich H oder D sind. Bevorzugt sind zwei Reste R ungleich H oder D. Bevorzugte Strukturen gemäß Formel (2), (3) sind die im Folgenden abgebildeten Strukturen gemäß Formel (6) bis (9).

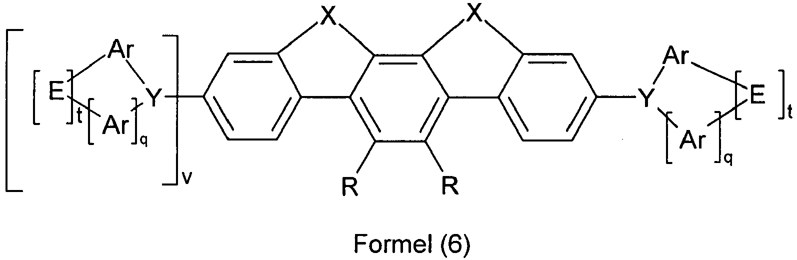

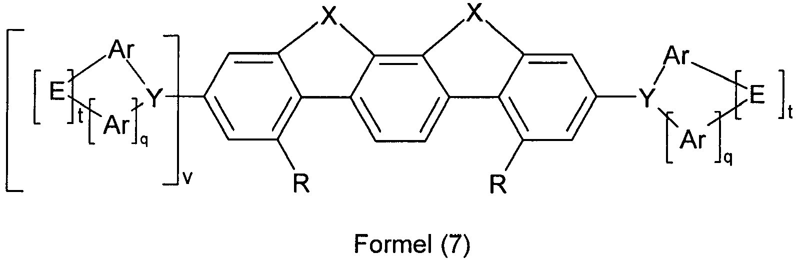

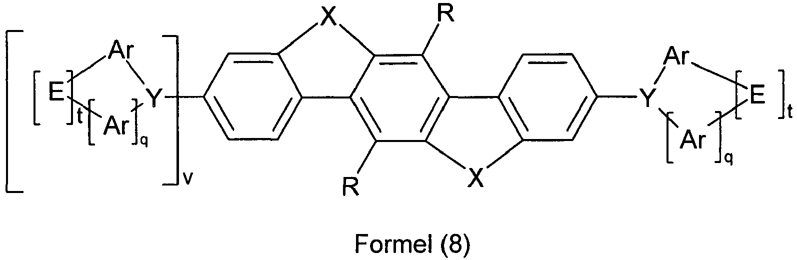

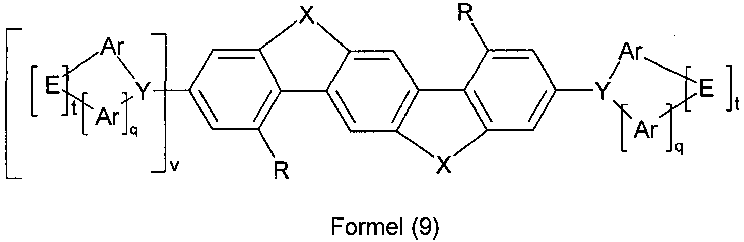

Dabei haben die Symbole und Indizes die oben genannten Bedeutungen und die Reste R sind ungleich Wasserstoff oder Deuterium. Bevorzugte Alkylgruppen als Reste R sind Methyl, Ethyl, n-Propyl, i-Propyl, n-Butyl, i-Butyl, s-Butyl, t-Butyl, 2-Butyl, 2-Methylbutyl, n-Pentyl, s-Pentyl, tert-Penyl, 2-Pentyl, 3-Pentyl, Cyclopentyl, n-Hexyl, s-Hexyl, tert-Hexyl, 2-Hexyl, 3-Hexyl, Cyclohexyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]octyl, 2-(2,6-Dimethyl)octyl oder 3-(3,7-Dimethyl)octyl. Wenn die Alkylgruppen chiral sind, so können sie entweder als enantiomerenreine Gruppe bzw. diastereomerenreine Gruppe verwendet werden oder als Mischung der Enantiomeren, insbesondere als Racemat, oder Mischung der Diastereomeren. Insbesondere für Verbindungen, welche bei der Device-Herstellung aus der Gasphase aufgedampft werden, ist die Alkylgruppe ganz besonders bevorzugt Methyl.

Bevorzugt sind weiterhin Verbindungen gemäß Formel (1) bis (9), in denen die Symbole X gleich C(R¹)₂ sind.

Ganz besonders bevorzugt sind daher Verbindungen der Formeln (6a) bis (9a), 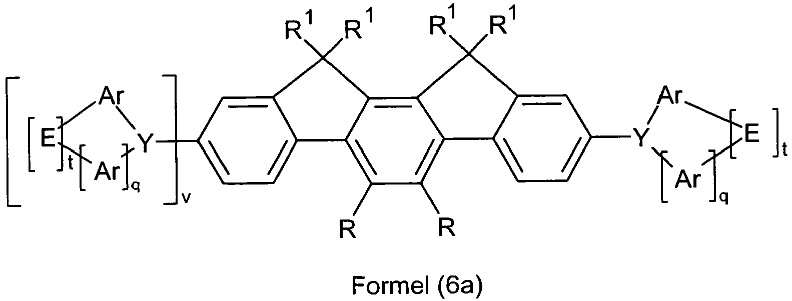 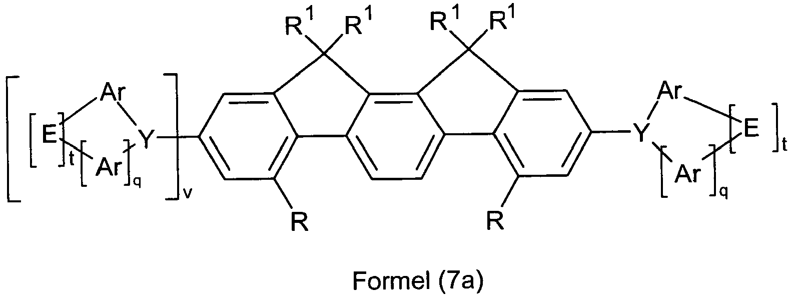 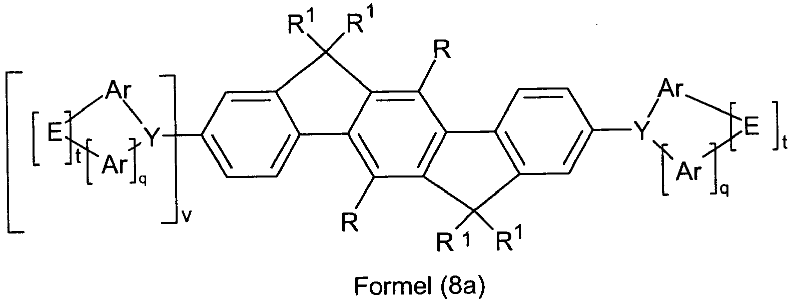 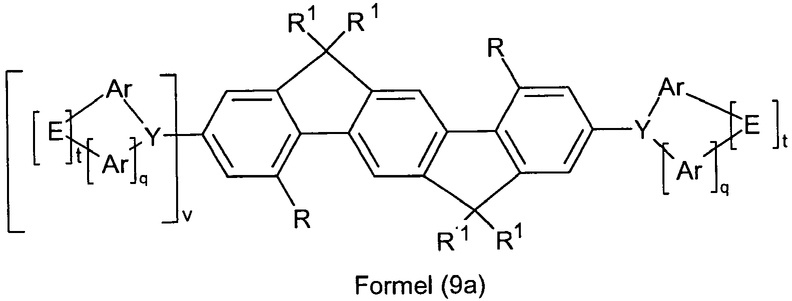 wobei die verwendeten Symbole und Indizes die oben genannten Bedeutungen haben und R ungleich Wasserstoff und ungleich Deuterium ist.

Bevorzugt sind weiterhin Verbindungen gemäß Formel (1) bis (9) bzw. (6a) bis (9a), in denen die Symbole Y gleich gewählt sind. Ganz besonders bevorzugt sind Verbindungen, in denen zusätzlich, falls vorhanden, beide Gruppen E gleich gewählt sind.

In den vorherigen Ausführungen sind jeweils die bevorzugten Ausführungsformen für die verwendeten Symbole und Indizes aufgeführt. Besonders bevorzugt sind Verbindungen gemäß Formel (1) bis (9) bzw. (6a) bis (9a), in denen die oben aufgeführten bevorzugten Ausführungsformen miteinander kombiniert werden.

Beispiele für bevorzugte Verbindungen gemäß Formel (1) sind die im Folgenden abgebildeten Strukturen.

| | |
|---|---|
| 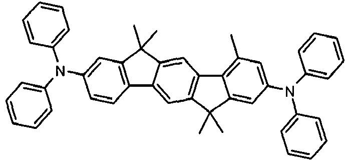 | 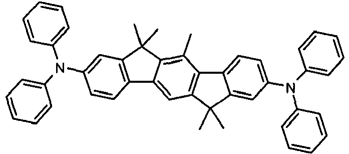 |
| (1) | (2) |
| 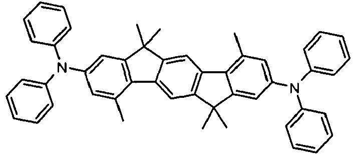 | 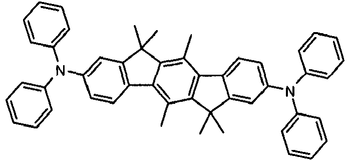 |
| (3) | (4) |
| 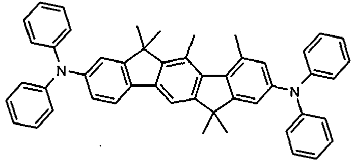 | 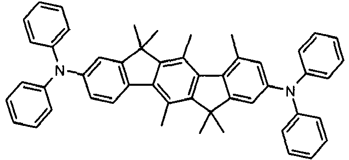 |
| (5) | (6) |
| 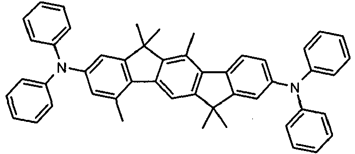 | 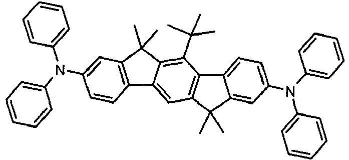 |
| (7) | (12) |
| 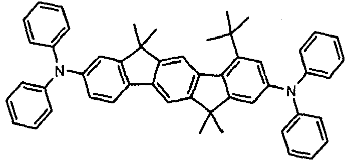 | 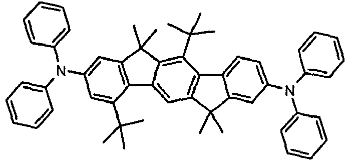 |
| (13) | (14) |
| 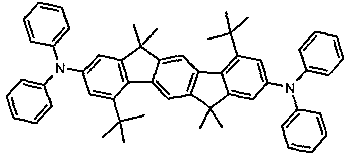 | 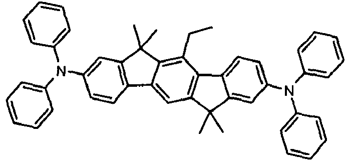 |
| (15) | (16) |
| 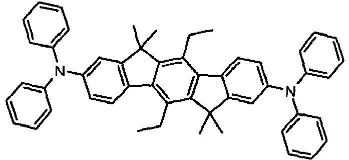 | 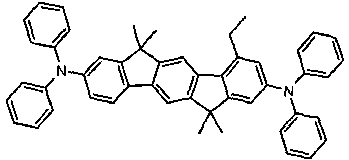 |
| (17) | (18) |
| 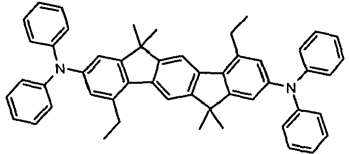 | 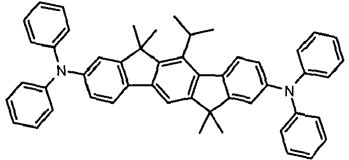 |
| (19) | (20) |
| 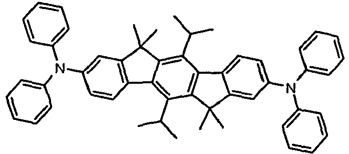 | 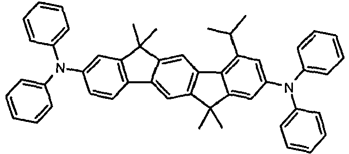 |
| (21) | (22) |
| 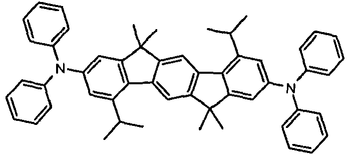 | 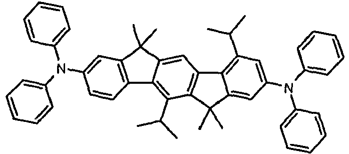 |
| (23) | (24) |
| 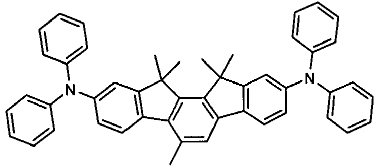 | 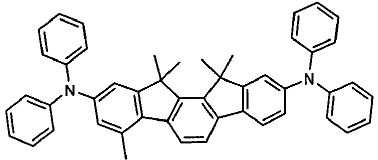 |
| (25) | (26) |
| 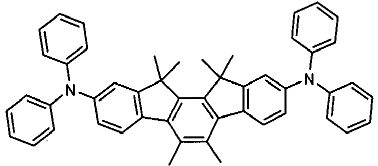 | 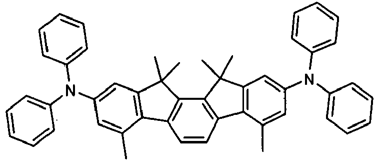 |
| (27) | (28) |
| 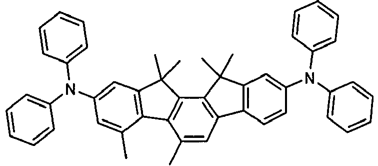 | 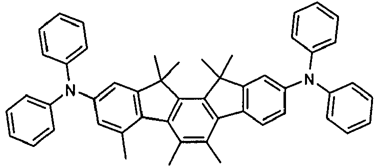 |
| (29) | (30) |
| 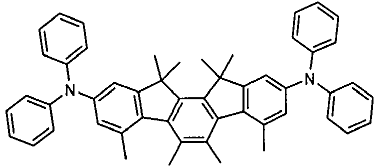 | |
| (31) | |
| | 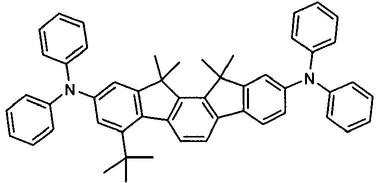 |
| | (36) |
| 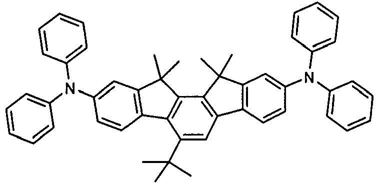 | 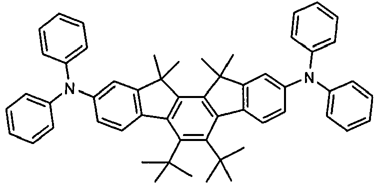 |
| (37) | (38) |
| 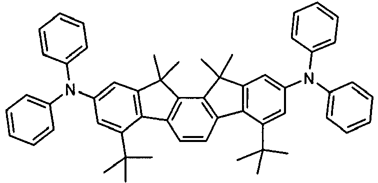 | 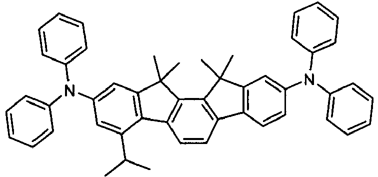 |
| (39) | (40) |
| 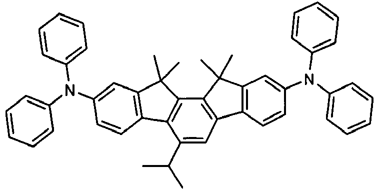 | 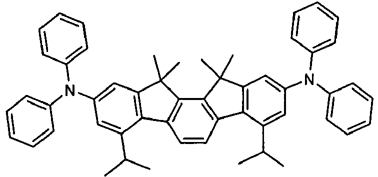 |
| (41) | (42) |
| 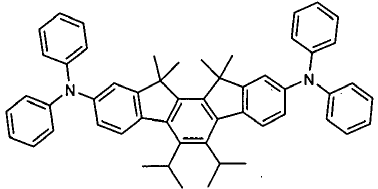 | 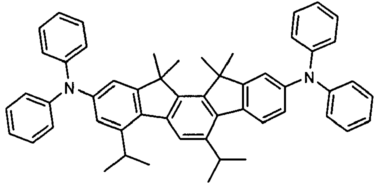 |
| (43) | (44) |
| 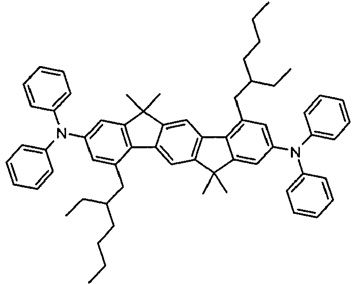 | 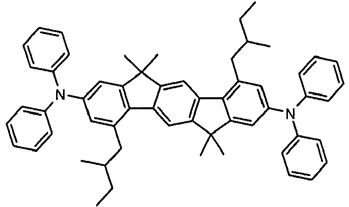 |
| (65) | (66) |
| 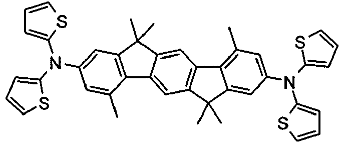 | 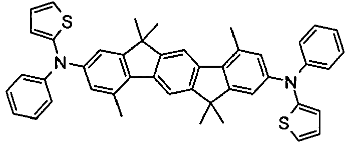 |
| (81) | (84) |
| | 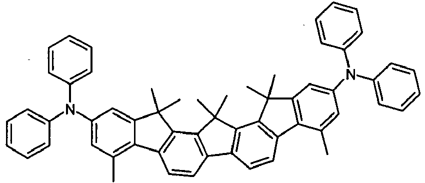 |
| | (88) |
| 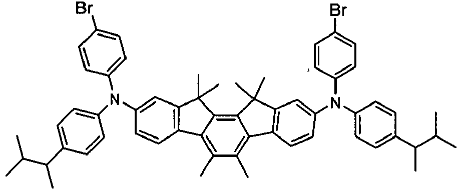 | 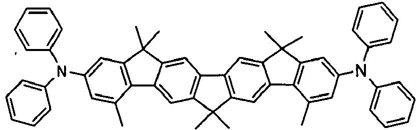 |
| (89) | (90) |
| 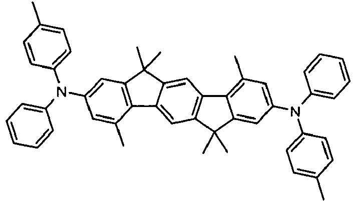 | 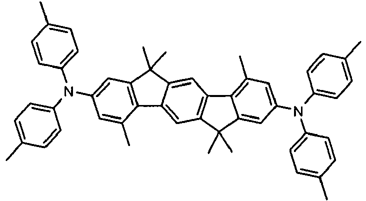 |
| (107) | (108) |
| 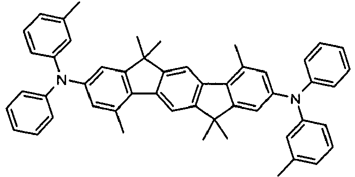 | 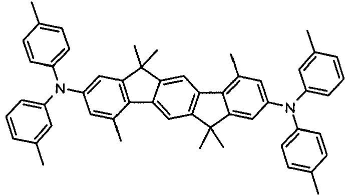 |
| (109) | (110) |
| 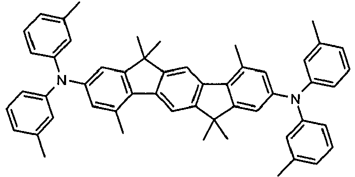 | 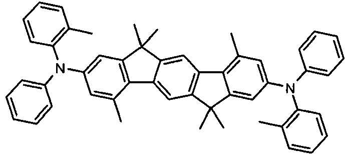 |
| (111) | (112) |
| 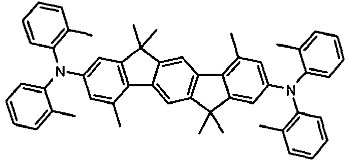 | 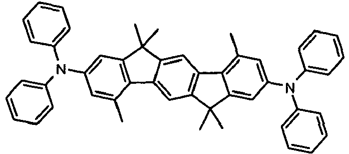 |
| (113) | (114) |
| 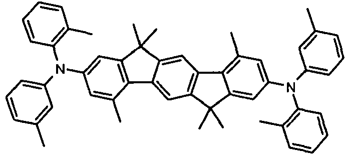 | 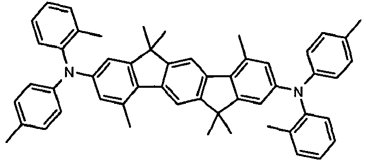 |
| (115) | (116) |
| 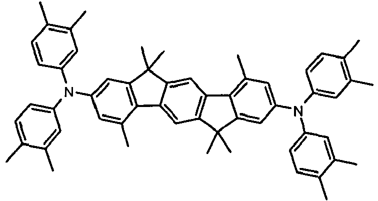 | 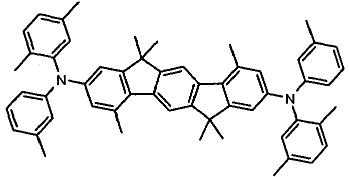 |
| (117) | (118) |
| 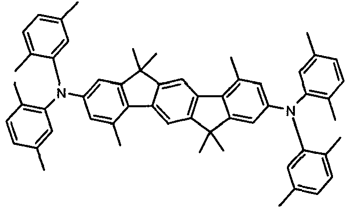 | 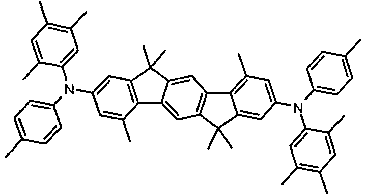 |
| (119) | (120) |
| 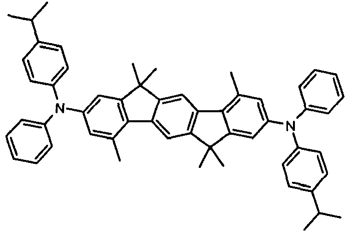 | 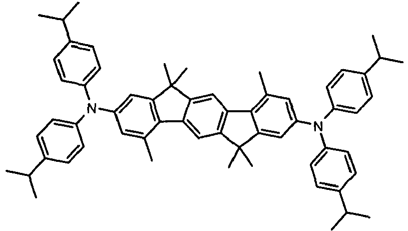 |
| (121) | (122) |
| 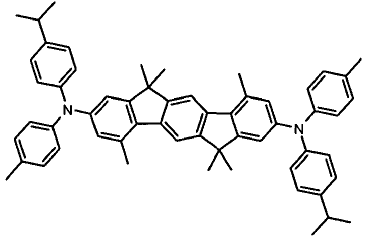 | 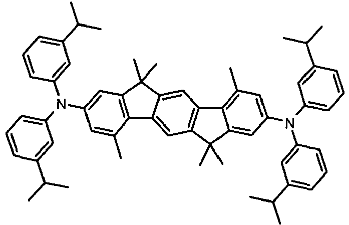 |
| (123) | (124) |
| 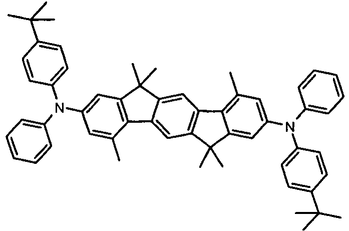 | 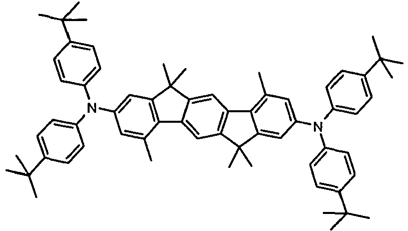 |
| (125) | (126) |
| 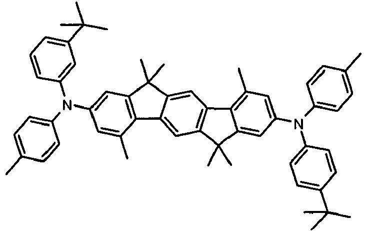 | 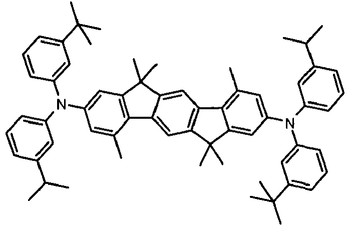 |
| (127) | (128) |
| 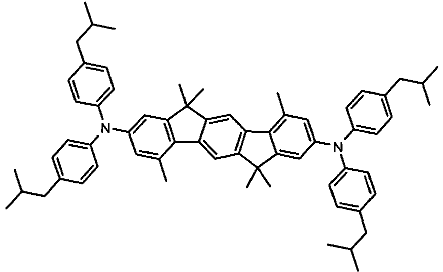 | 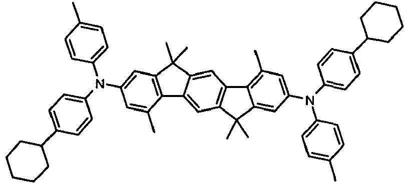 |
| (129) | (130) |
| 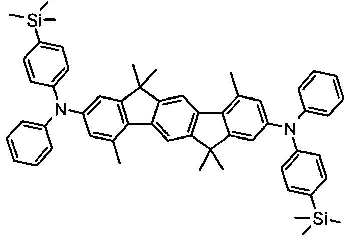 | 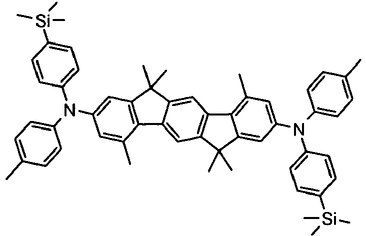 |
| (131) | (132) |
| 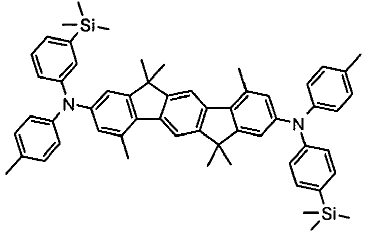 | 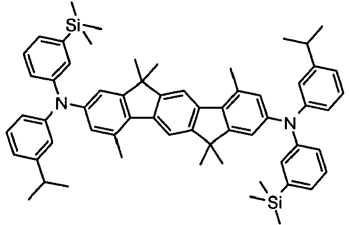 |
| (133) | (134) |
| 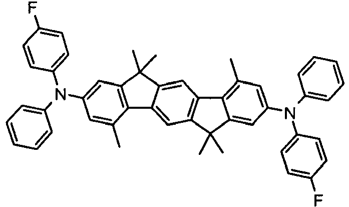 | 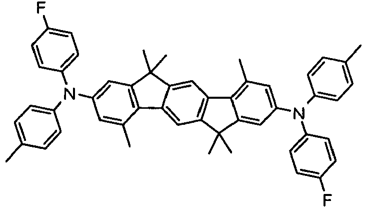 |
| (135) | (136) |
| 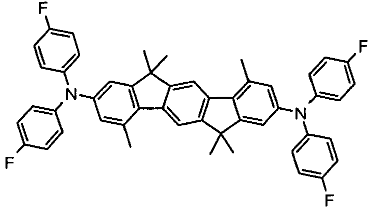 | 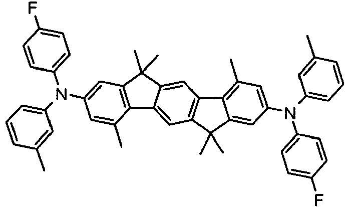 |
| (137) | (138) |
| 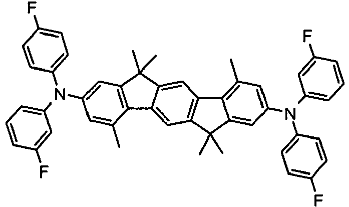 | 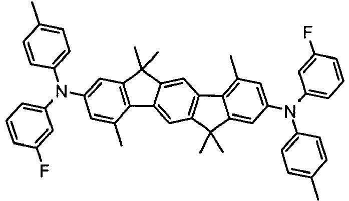 |
| (139) | (140) |
| 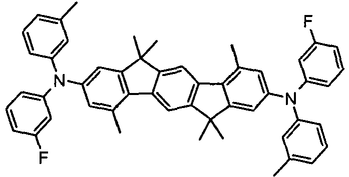 | 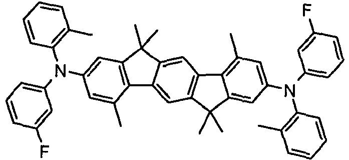 |
| (141) | (142) |
| 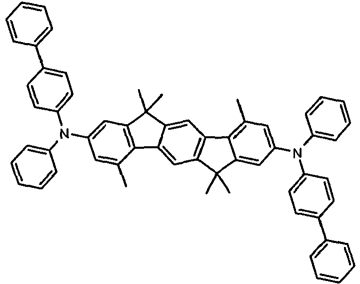 | 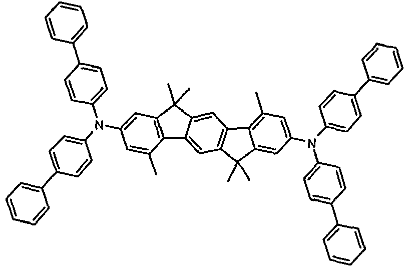 |
| (143) | (144) |
| 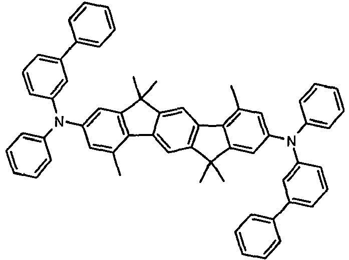 | 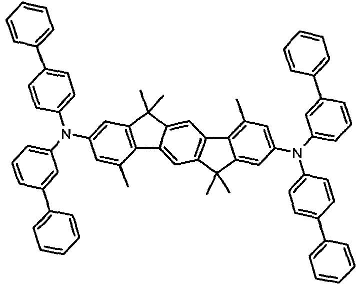 |
| (145) | (146) |
| 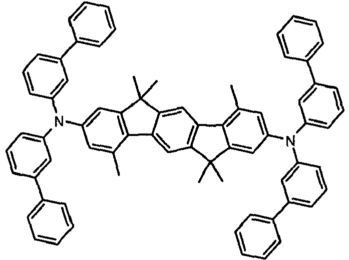 | 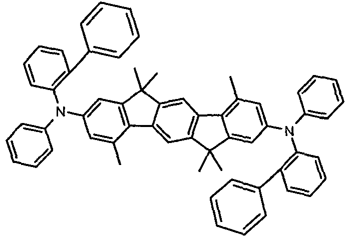 |
| (147) | (148) |
| 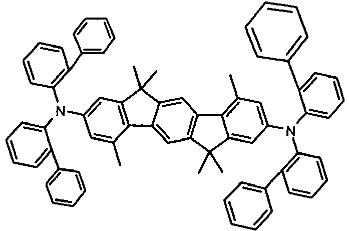 | 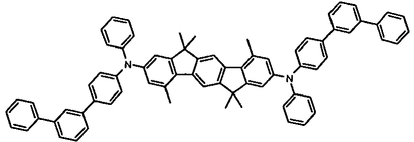 |
| (149) | (150) |
| 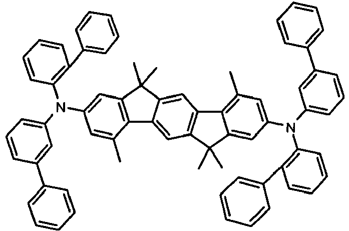 | 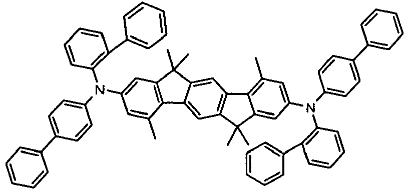 |
| (151) | (152) |
| 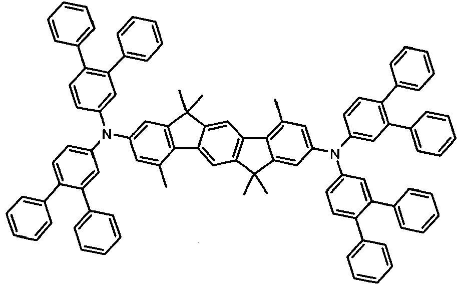 | 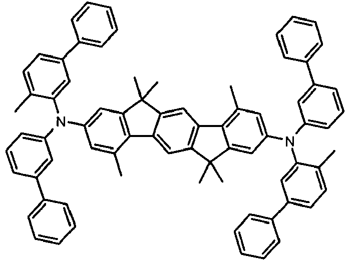 |
| (153) | (154) |
| 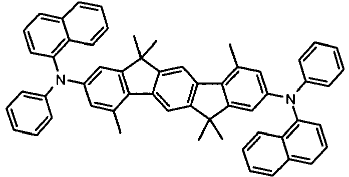 | 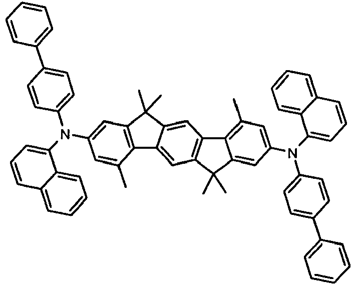 |
| (155) | (156) |
| 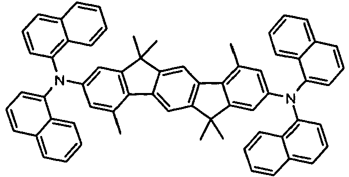 | 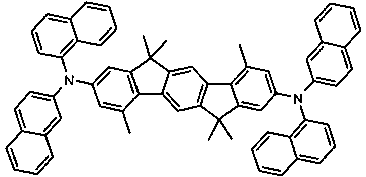 |
| (157) | (158) |
| 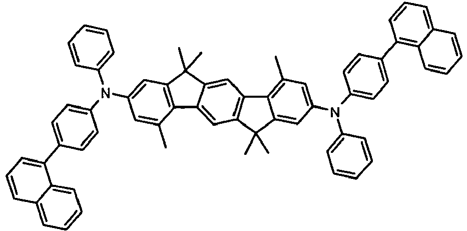 | 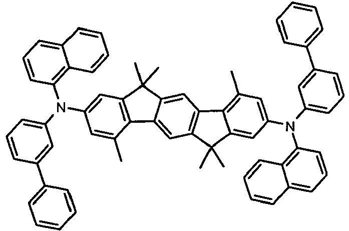 |
| (159) | (160) |
| 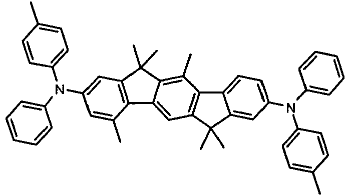 | 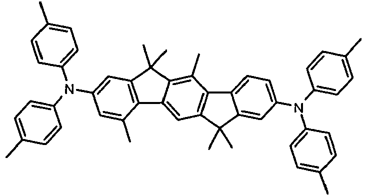 |
| (161) | (162) |
| 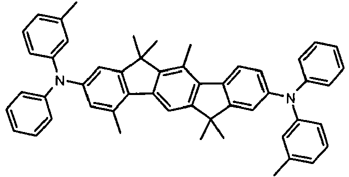 | 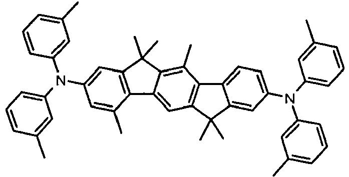 |
| (163) | (164) |
| 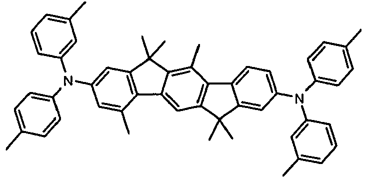 | 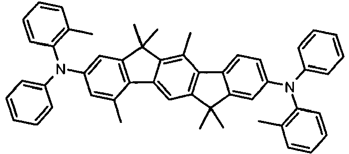 |
| (165) | (166) |
| 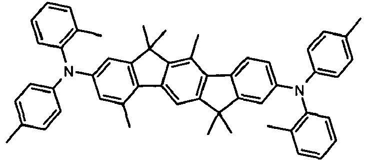 | 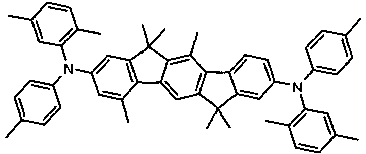 |
| (167) | (168) |
| 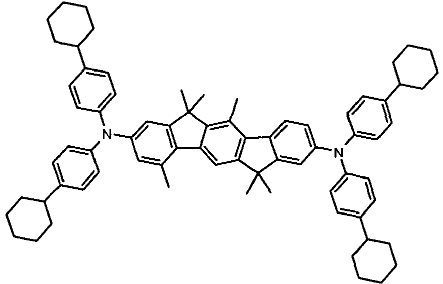 | 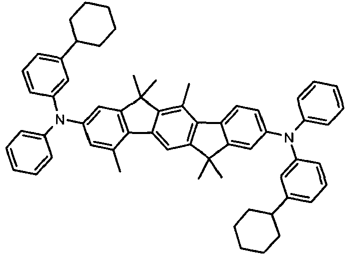 |
| (169) | (170) |
| 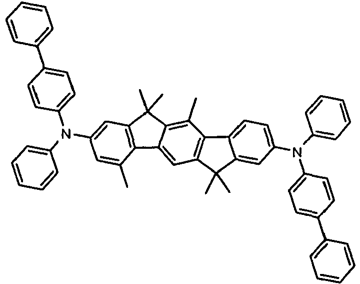 | 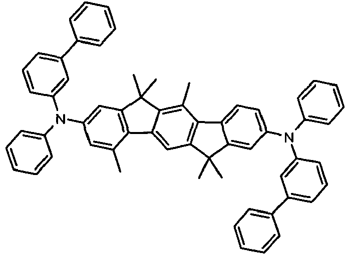 |
| (171) | (172) |
| 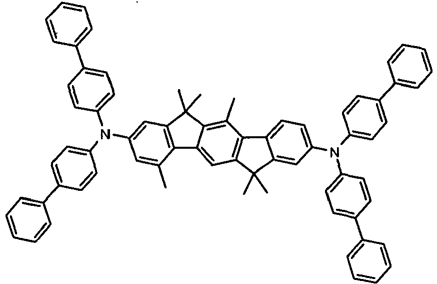 | 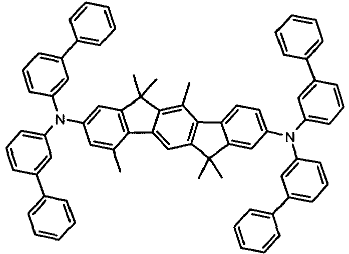 |
| (173) | (174) |
| 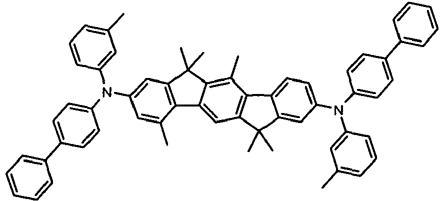 | 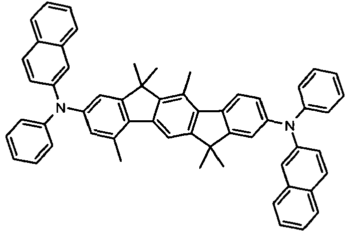 |
| (175) | (176) |
| 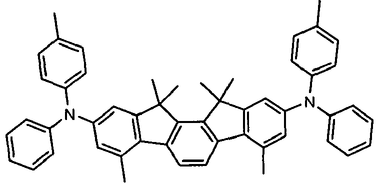 | 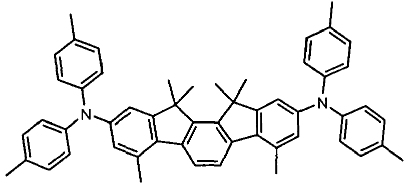 |
| (177) | (178) |
| 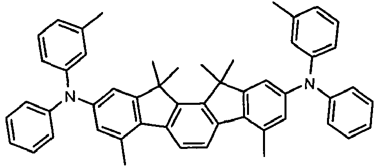 | 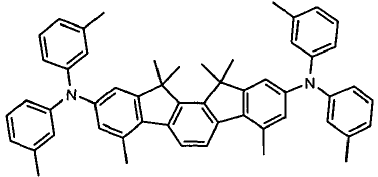 |
| (179) | (180) |
| 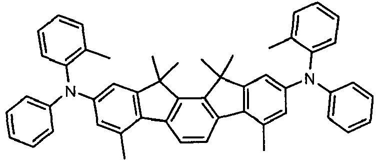 | 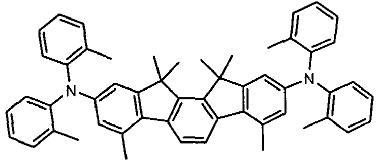 |
| (181) | (182) |
| 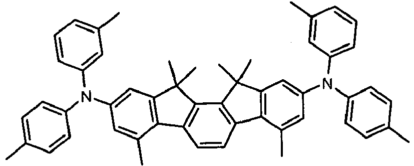 | 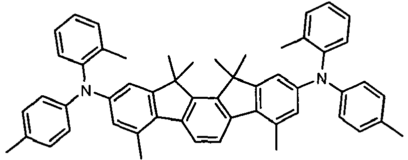 |
| (183) | (184) |
| 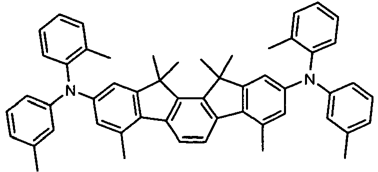 | 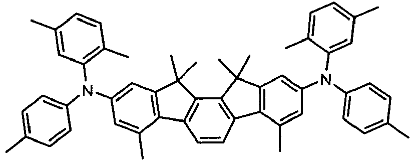 |
| (185) | (186) |
| 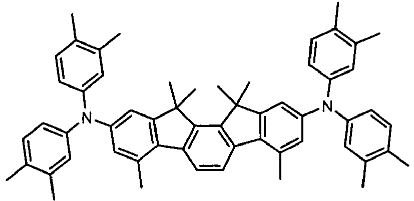 | 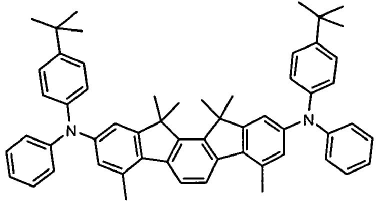 |
| (187) | (188) |
| 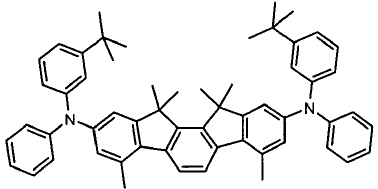 | 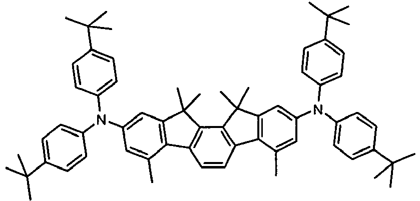 |
| (189) | (190) |
| 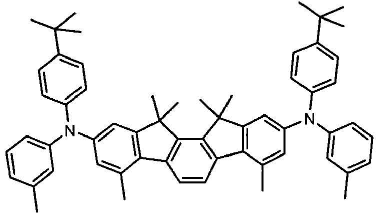 | 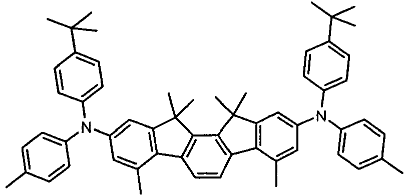 |
| (191) | (192) |
| 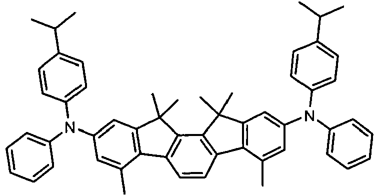 | 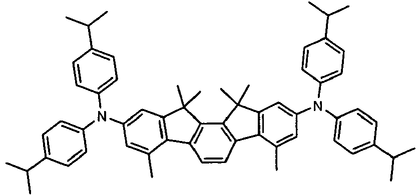 |
| (193) | (194) |
| 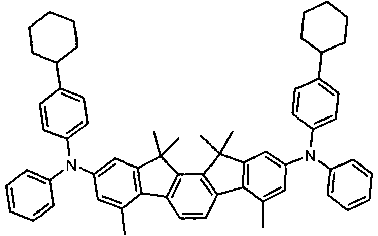 | 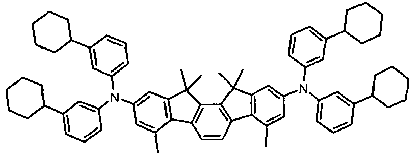 |
| (195) | (196) |
| 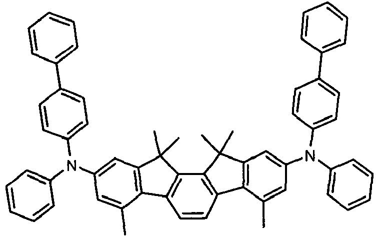 | 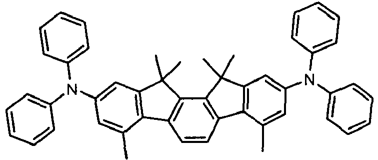 |
| (197) | (198) |
| 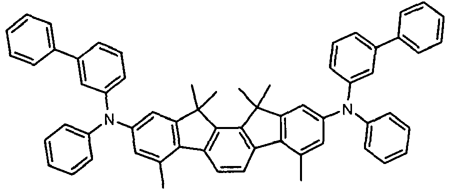 | 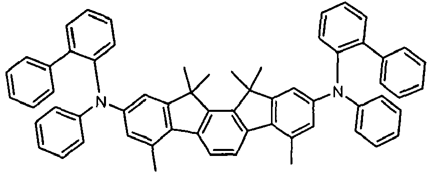 |
| (199) | (200) |
| 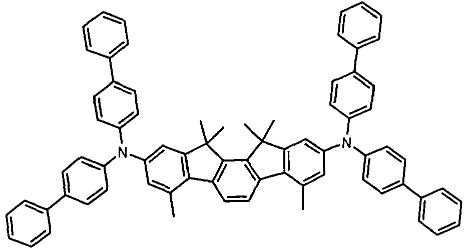 | 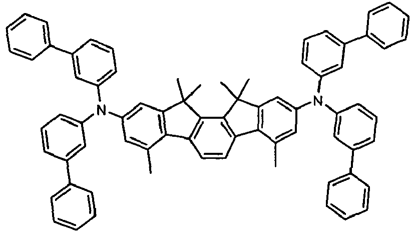 |
| (201) | (202) |
| 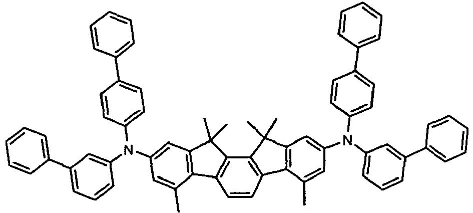 | 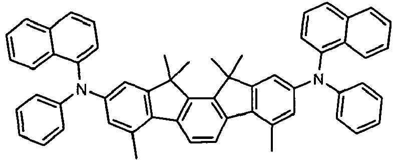 |
| (203) | (204) |
| 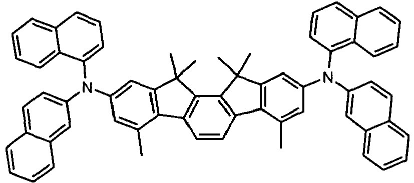 | 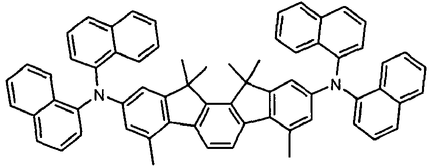 |
| (205) | (206) |
| 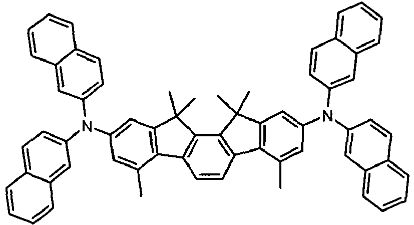 | 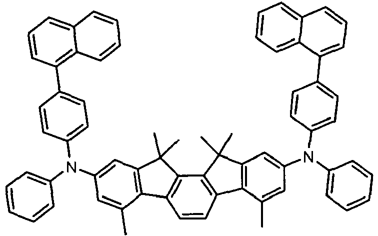 |
| (207) | (208) |
| 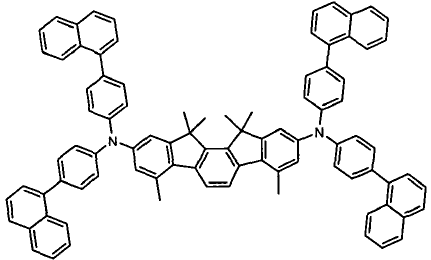 | 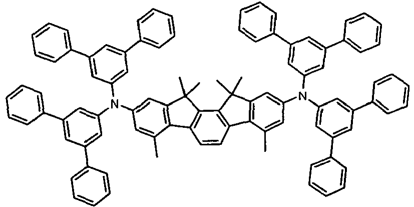 |
| (209) | (210) |
| 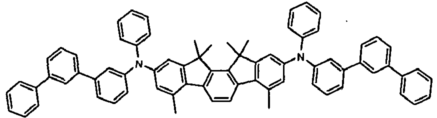 | 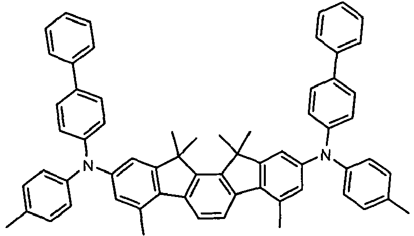 |
| (211) | (212) |
| 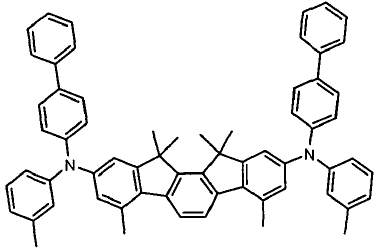 | 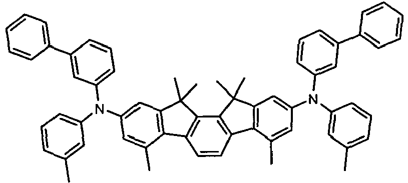 |
| (213) | (214) |
| 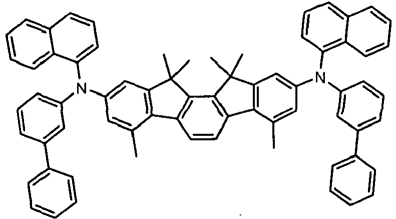 | 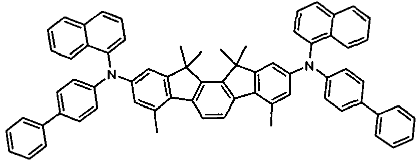 |
| (215) | (216) |
| 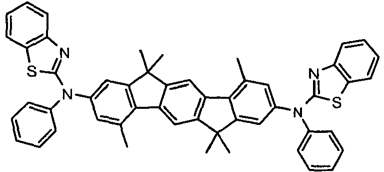 | 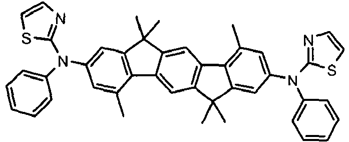 |
| (217) | (218) |
| 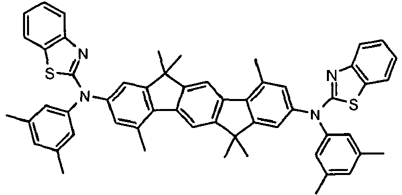 | 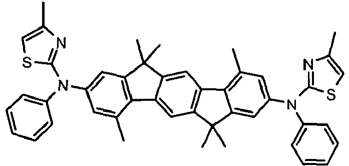 |
| (219) | (220) |
| 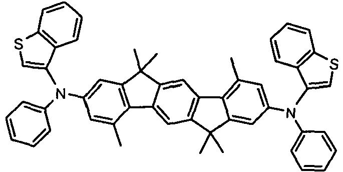 | 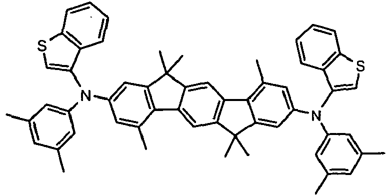 |
| (221) | (222) |
| 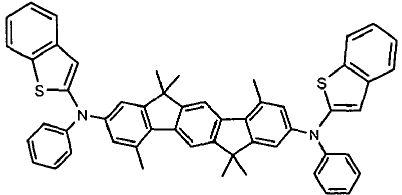 | 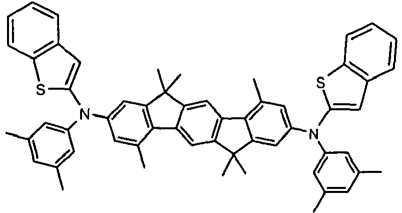 |
| (223) | (224) |
| 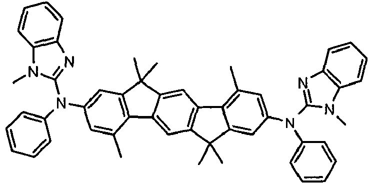 | 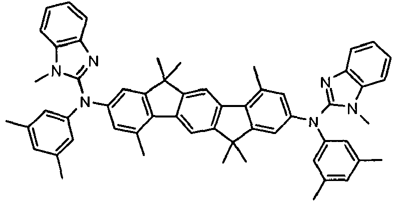 |
| (225) | (226) |
| 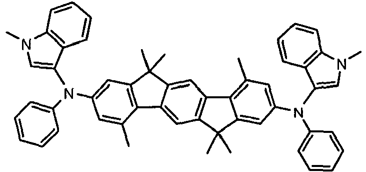 | 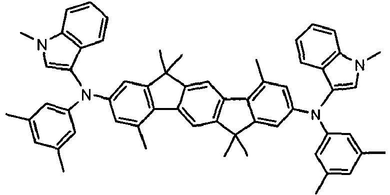 |
| (227) | (228) |
| 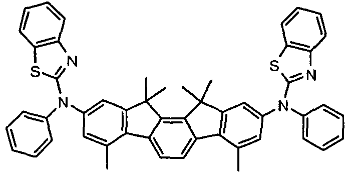 | 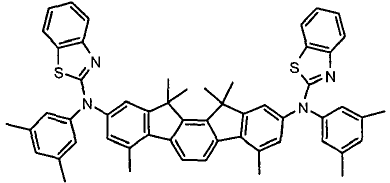 |
| (229) | (230) |
| 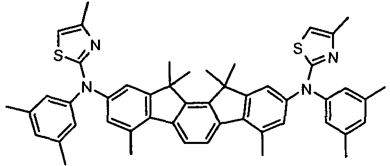 | 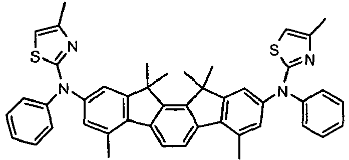 |
| (231) | (232) |
| 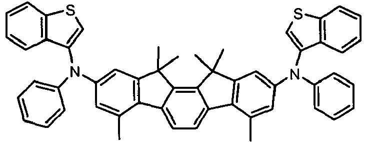 | 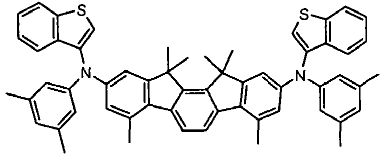 |
| (233) | (234) |
| 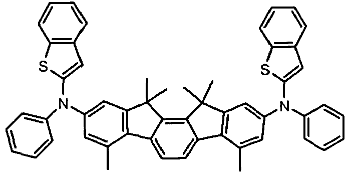 | 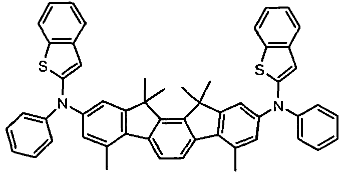 |
| (235) | (236) |
| 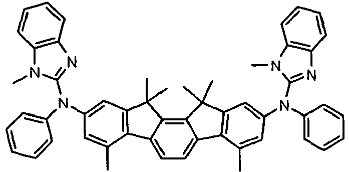 | 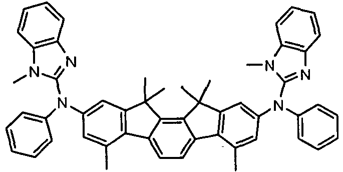 |
| (237) | (238) |
| 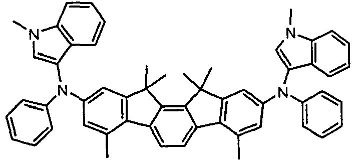 | 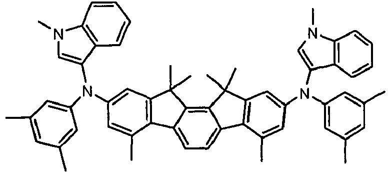 |
| (239) | (240) |
| 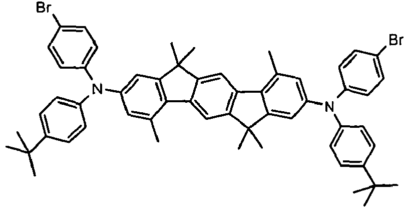 | 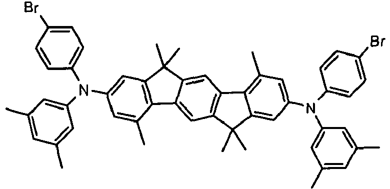 |
| (241) | (242) |
| 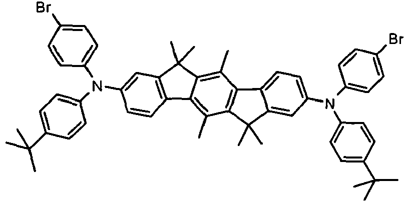 | 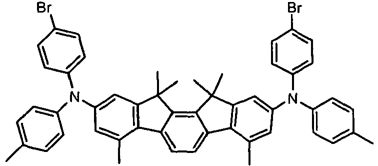 |
| (243) | (244) |
| 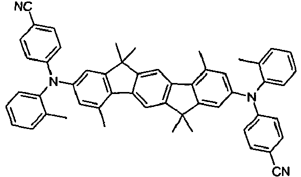 | 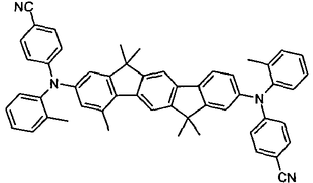 |
| (245) | (246) |
| 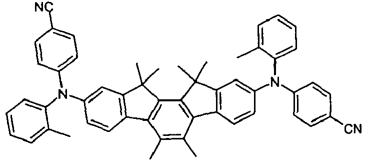 | 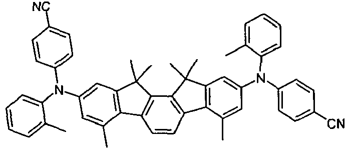 |
| (247) | (248) |
| 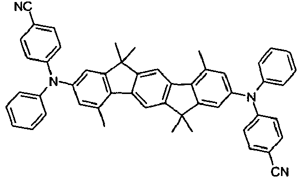 | 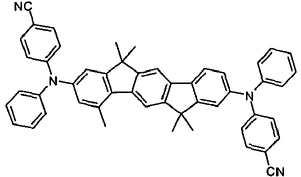 |
| (249) | (250) |
| 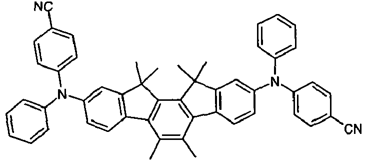 | 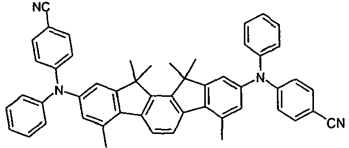 |
| (251) | (252) |
| 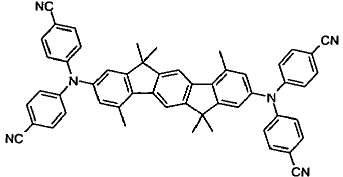 | 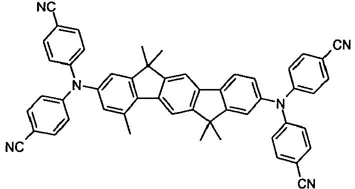 |
| (253) | (254) |
| 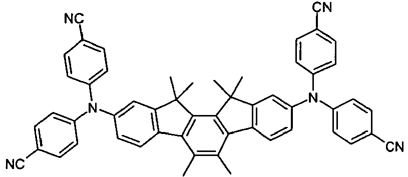 | 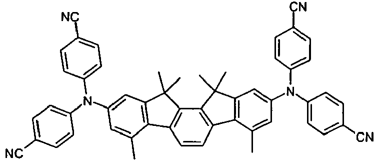 |
| (255) | (256) |
| 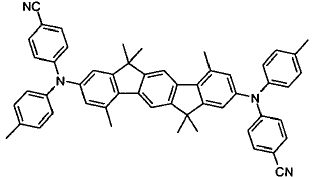 | 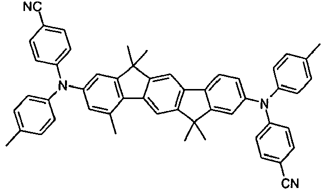 |
| (257) | (258) |
| 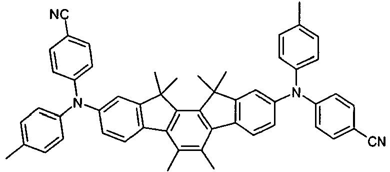 | 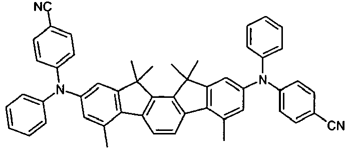 |
| (259) | (260) |
| 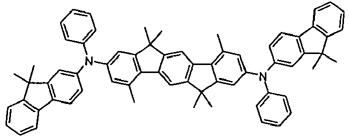 | 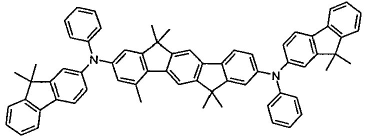 |
| (261) | (262) |
| 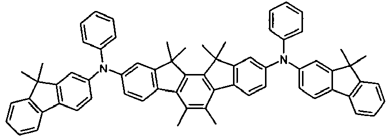 | 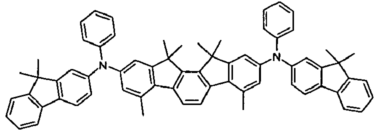 |
| (263) | (264) |
| 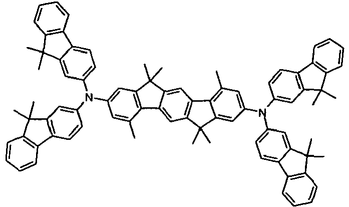 | 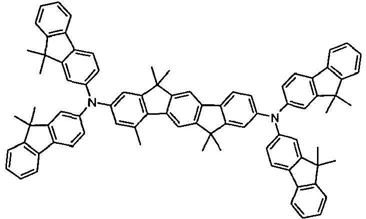 |
| (265) | (266) |
| 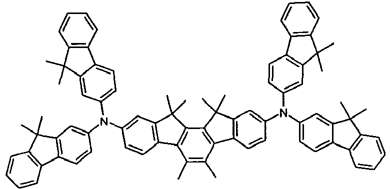 | 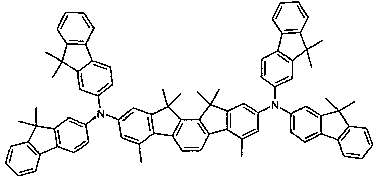 |
| (267) | (268) |
| 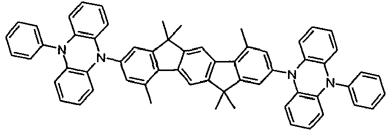 | 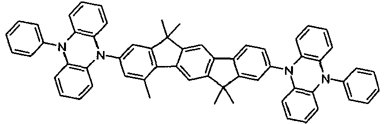 |
| (269) | (270) |
| 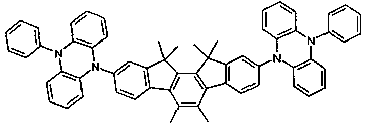 | 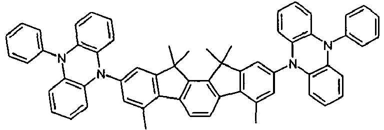 |
| (271) | (272) |
| 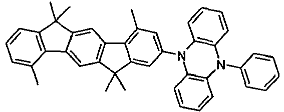 | 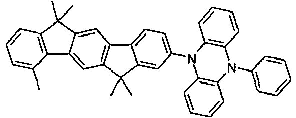 |
| (273) | (274) |
| 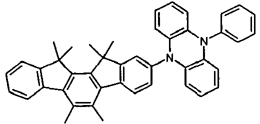 | 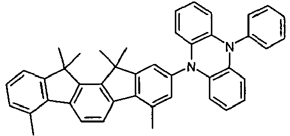 |
| (275) | (276) |
| 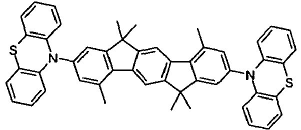 | 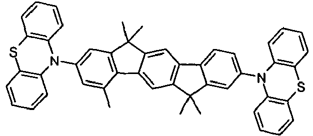 |
| (277) | (278) |
| 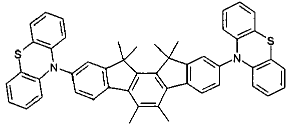 | 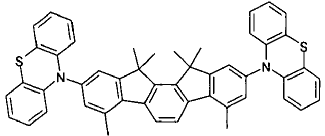 |
| (279) | (280) |
| 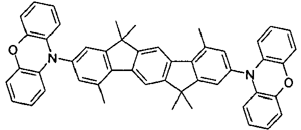 | 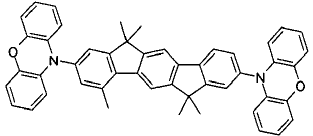 |
| (281) | (282) |
| 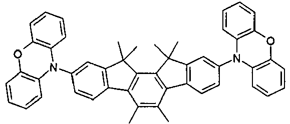 | 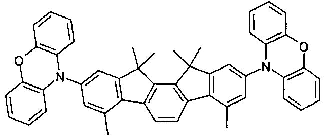 |
| (283) | (284) |
| 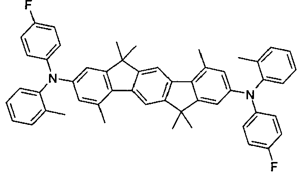 | 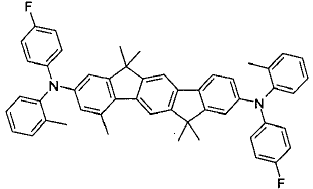 |
| (289) | (290) |
| 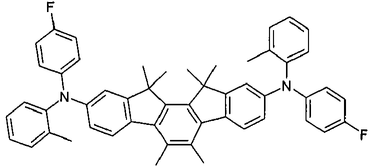 | 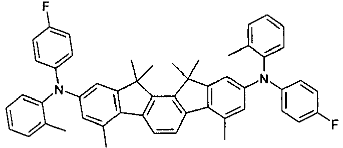 |
| (291) | (292) |
| 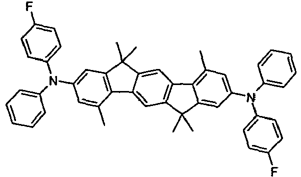 | 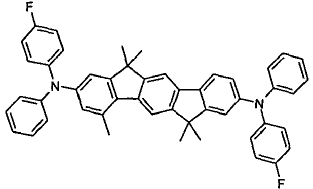 |
| (293) | (294) |
| 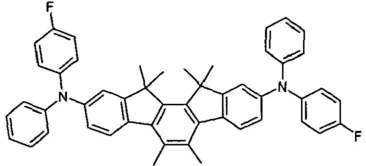 | 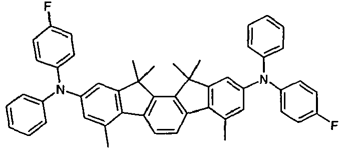 |
| (295) | (296) |
| 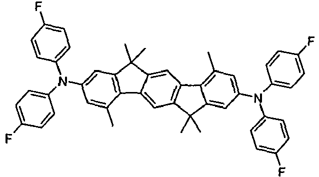 | 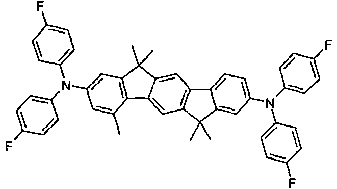 |
| (297) | (298) |
| 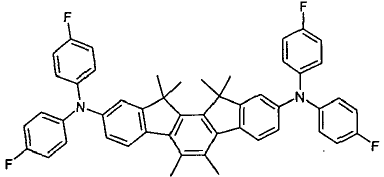 | 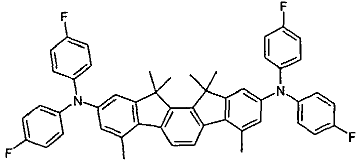 |
| (299) | (300) |
| 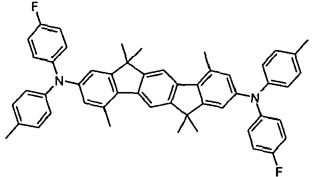 | 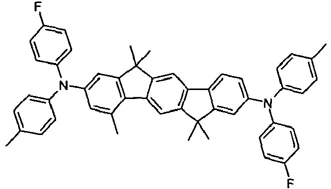 |
| (301) | (302) |
| 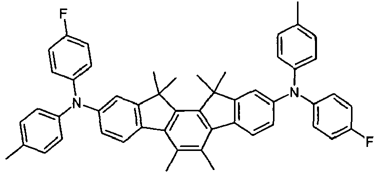 | 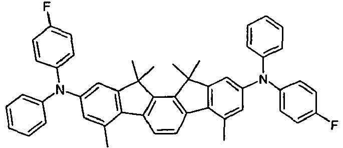 |
| (303) | (304) |
| 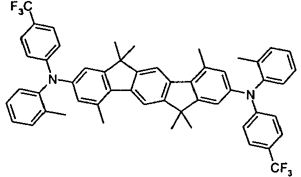 | 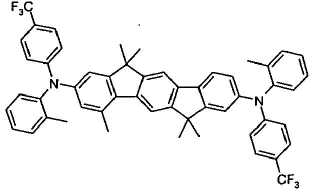 |
| (305) | (306) |
| 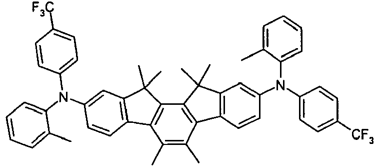 | 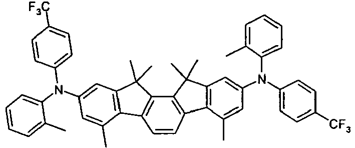 |
| (307) | (308) |
| 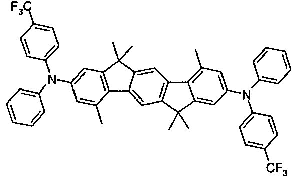 | 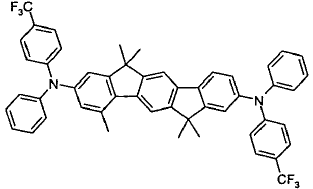 |
| (309) | (310) |
| 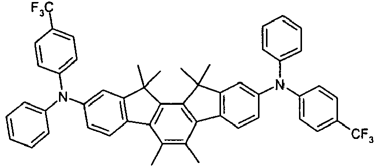 | 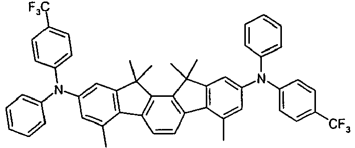 |
| (311) | (312) |
| 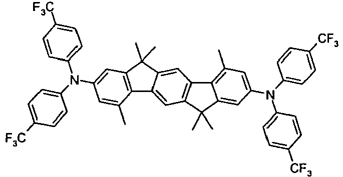 | 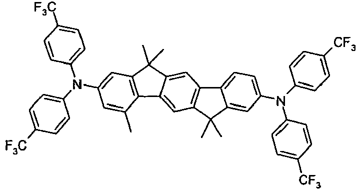 |
| (313) | (314) |
| 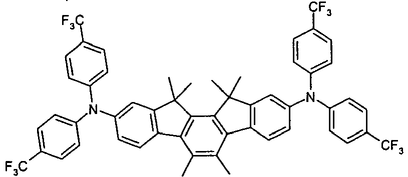 | 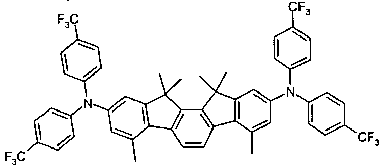 |
| (315) | (316) |
| 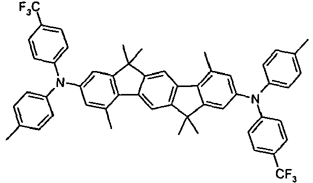 | 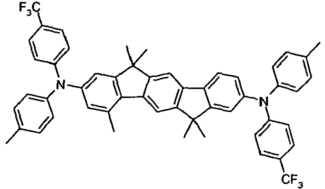 |
| (317) | (318) |
| 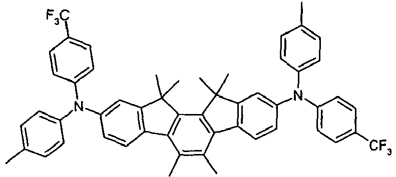 | 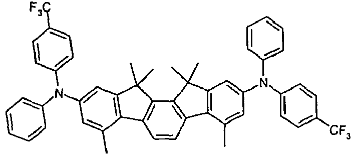 |
| (319) | (320) |

Die erfindungsgemäßen Verbindungen können nach dem Fachmann bekannten Syntheseschritten, wie z. B. Bromierung, Suzuki-Kupplung, Hartwig-Buchwald-Kupplung, etc., dargestellt werden. Verbindungen gemäß Formel (6a) können, wie in Syntheseschema 1 gezeigt, dargestellt werden. Ein entsprechend 2,3-substuiertes 1,4-Benzoldiboronsäurederivat wird mit einem 2-Brombenzoat unter Palladiumkatalyse gekuppelt. Ebenso können andere Kupplungsreaktionen verwendet werden. Nach einer Cyclisierung zum Indenofluoren kann dieses unter Standardbedingungen, wie sie dem Fachmann der organischen Chemie bekannt sind, bromiert werden. Die bromierten Verbindungen können durch eine Buchwald-Kupplung unter Standardbedingungen mit aromatischen Aminen gekuppelt werden.

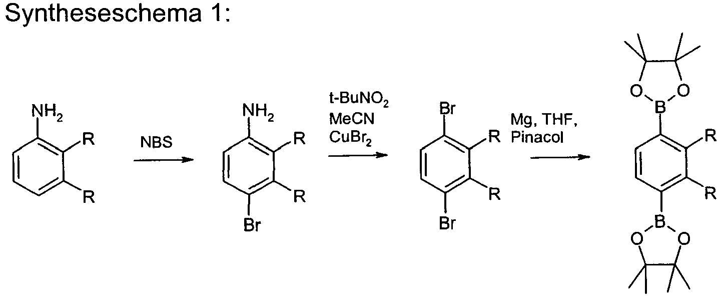

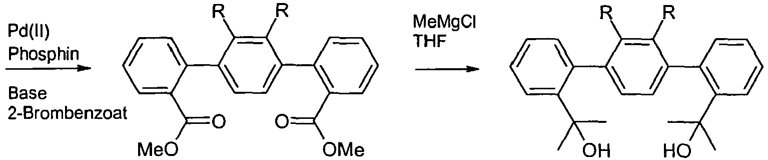

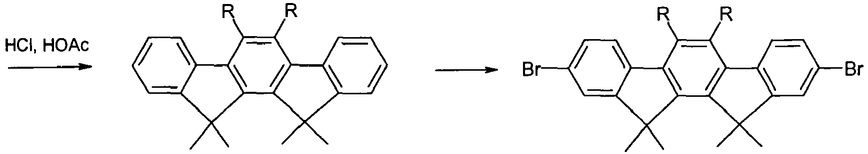

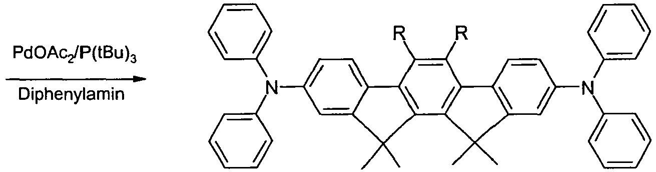

Die Synthese von Verbindungen gemäß Formel (7a) wird in Syntheseschema 2 gezeigt. Das substituierte cis-Indenofluoren-Grundgerüst kann durch Diels-Alder-Reaktion eines entsprechend substituierten Diphenylbutadienderivats mit einem Acetylendicarbonsäurederivat, gefolgt von Aromatisierung, Reduktion der Estergruppen und Cyclisierung zum Indenofluoren erhalten werden. Dieses kann, gegebenenfalls nach Substitution der Indenobrücken, unter Standardbedingungen, wie sie dem Fachmann der organischen Chemie bekannt sind, bromiert werden. Die bromierten Verbindungen können durch eine Buchwald-Kupplung unter Standardbedingungen mit aromatischen Aminen gekuppelt werden, wie in Syntheseschema 3 gezeigt.

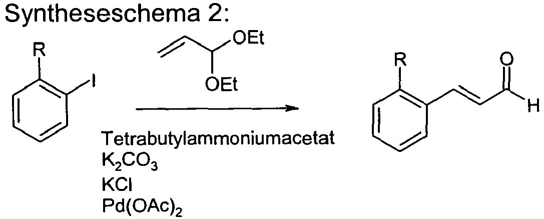

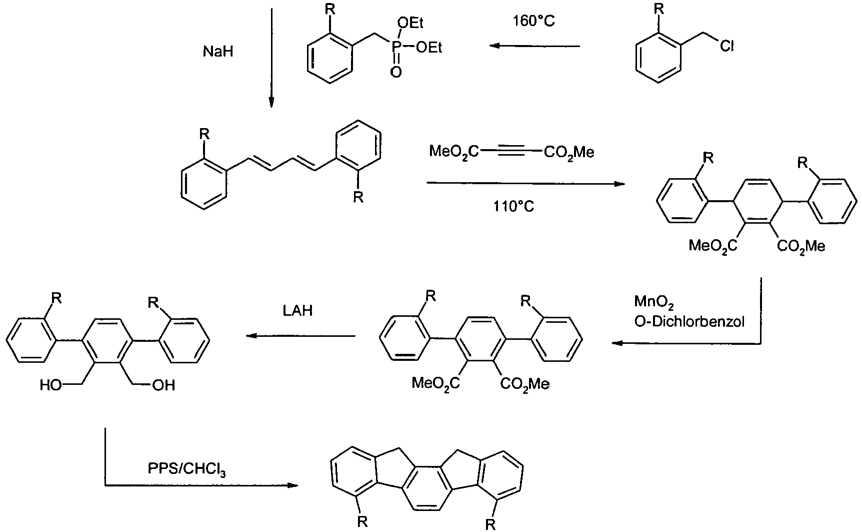

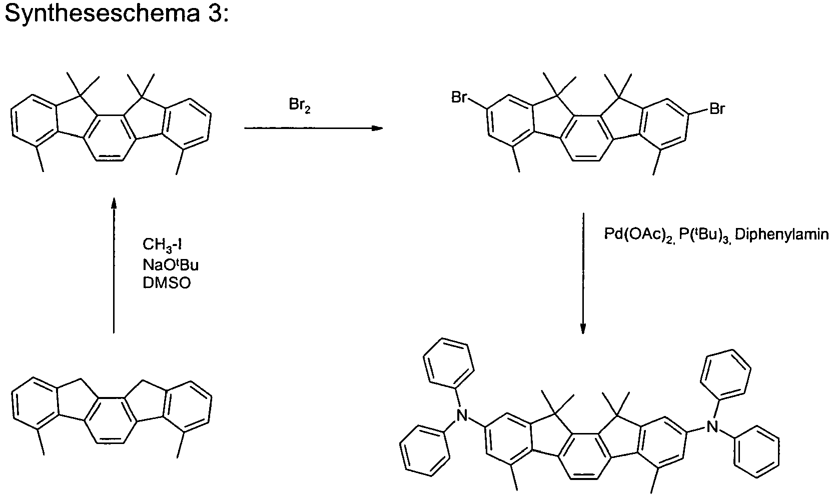

Ebenso eignen sich die bromierten Verbindungen als Zwischenstufe zu weiteren erfindungsgemäßen Derivaten. So kann die Synthese entsprechender Phosphine aus dem Dibromindenofluoren durch Lithiierung und Umsetzung mit Diarylchlorphosphinen erfolgen. Oxidation ergibt dann das entsprechende Phosphinoxid. Ebenso lassen sich hier andere Elektrophile einsetzen, wie z. B. AsCl₃, ArylPCl₂, SOCl₂, Ar₂S₂, etc. Weitere erfindungsgemäße Verbindungen können nach diesen und ähnlichen Syntheseschemata leicht in dem Fachmann für organische Synthese bekannten Verfahren synthetisiert werden.

Ein weiterer Gegenstand der vorliegenden Erfindung ist ein Verfahren zur Herstellung der erfindungsgemäßen Verbindungen, umfassend die Reaktionsschritte:
a) Synthese des Indenofluorengrundkörpers bzw. des entsprechenden heterocyclischen Derivats, welches statt der Gruppen Y in diesen Positionen Wasserstoff trägt;
b) Halogenierung, wobei ein Indenofluoren bzw. ein entsprechendes heterocyclisches Derivat entsteht, welches statt der Gruppen Y in diesen Positionen durch Halogen substituiert ist; und
c) Umsetzung des halogenierten Indenofluorens mit einem Diarylamin oder Metallierung und Umsetzung mit einem Elektrophil.

Die erfindungsgemäßen Verbindungen gemäß Formel (1) eignen sich für den Einsatz in elektronischen Vorrichtungen, insbesondere in organischen Elektrolumineszenzvorrichtungen (OLEDs, PLEDs). Abhängig von der Substitution werden die Verbindungen in unterschiedlichen Funktionen und Schichten eingesetzt. Die genaue Verwendung der Verbindungen hängt dabei insbesondere von der Wahl der Gruppen Y, aber auch von der Wahl der Gruppen X ab.

Ein weiterer Gegenstand der Erfindung ist daher die Verwendung der erfindungsgemäßen Verbindungen gemäß Formel (1) in elektronischen Vorrichtungen, insbesondere in organischen Elektrolumineszenzvorrichtungen.

Nochmals ein weiterer Gegenstand der Erfindung sind organische elektronische Vorrichtungen, enthaltend mindestens eine Verbindung gemäß Formel (1), insbesondere organische Elektrolumineszenzvorrichtungen, enthaltend Anode, Kathode und mindestens eine emittierende Schicht, dadurch gekennzeichnet, dass mindestens eine organische Schicht, die eine emittierende Schicht oder eine andere Schicht sein kann, mindestens eine Verbindung gemäß Formel (1) enthält. Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Exzitonenblockierschichten, Charge-Generation Layers (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

Die organische Elektrolumineszenzvorrichtung kann auch mehrere emittierende Schichten enthalten, wobei mindestens eine organische Schicht mindestens eine Verbindung gemäß Formel (1) enthält. Besonders bevorzugt weisen diese Emissionsschichten insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues und gelbes, orange oder rotes Licht emittieren. Insbesondere bevorzugt sind Dreischichtsysteme, also Systeme mit drei emittierenden Schichten, wobei mindestens eine dieser Schichten mindestens eine Verbindung gemäß Formel (1) enthält und wobei die drei Schichten blaue, grüne und orange oder rote Emission zeigen (für den prinzipiellen Aufbau siehe z. B. WO 05/011013). Ebenso eignen sich für weiße Emission Emitter, welche breitbandige Emissionsbanden aufweisen und dadurch weiße Emission zeigen.

Es ist insbesondere bevorzugt, wenn die Verbindungen gemäß Formel (1) als Lochtransportmaterial und/oder als Lochinjektionsmaterial und/oder als Elektronenblockiermaterial eingesetzt werden.

Die Verbindungen werden dann bevorzugt in einer Lochtransportschicht und/oder in einer Lochinjektionsschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen der Lochinjektionsschicht und der Emissionsschicht liegt. Wenn die Verbindungen gemäß Formel (1) als Lochtransport- bzw. als Lochinjektionsmaterial verwendet werden, kann es bevorzugt sein, wenn sie mit Elektronenakzeptor-Verbindungen dotiert ist, beispielsweise mit F₄-TCNQ oder mit Verbindungen, wie sie in EP 1476881 oder EP 1596445 beschrieben werden. Wird die Verbindung gemäß Formel (1) als Lochtransportmaterial in einer Lochtransportschicht eingesetzt, kann auch ein Anteil von 100 % bevorzugt sein, also die Verwendung dieser Verbindung als Reinmaterial.

Es ist weiterhin bevorzugt, die Verbindungen der Formel (1) als Elektronentransportmaterial und/oder als Lochblockiermaterial für fluoreszierende und phosphoreszierende OLEDs und/oder als Triplett-Matrixmaterial für phosphoreszierende OLEDs einzusetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruckvon üblicherweise kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (1) nötig. Hohe Löslichkeit lässt sich durch geeignete Substitution der Verbindungen erreichen.

Die erfindungsgemäßen Verbindungen weisen bei Verwendung in organischen Elektrolumineszenzvorrichtungen folgende überraschende Vorteile gegenüber dem Stand der Technik auf:
1. Bei Verwendung der erfindungsgemäßen Verbindungen als Lochtransportmaterial in einer Lochtransport- und/oder Lochinjektionsschicht werden geringere Einsatzspannungen benötigt im Vergleich zu Verbindungen gemäß dem Stand der Technik, welche keine Substituenten R in einer der Positionen wie an den erfindungsgemäßen Verbindungen enthalten bzw. welche keinen Stickstoff im Indenofluoren-Grundgerüst enthalten.
2. Weiterhin ist bei Verwendung der erfindungsgemäßen Verbindungen als Lochtransportmaterial in einer Lochtransport- und/oder Lochinjektionsschicht die Betriebsspannung der organischen Elektrolumineszenzvorrichtung erheblich geringer ist als bei Verwendung von Verbindungen gemäß dem Stand der Technik. Daher führt die Verwendung der erfindungsgemäßen Verbindungen zu einer deutlich höheren Leistungseffizienz der OLED.
3. Ein weiterer Vorteil bei Verwendung der erfindungsgemäßen Verbindungen als Lochtransportmaterial in einer Lochtransport- und/oder Lochinjektionsschicht ist die verringerte Spannungsdifferenz zwischen dünnen (z. B. 20 nm) und dicken (z. B. 110 nm) Lochtransportschichten. Dadurch können mit den erfindungsgemäßen Verbindungen dickere Lochtransportschichten ohne einen erheblichen Verlust der Leistungseffizienz verwendet werden. Dies ist wichtig, da die optische Auskoppeleffizienz maßgeblich durch eine Variation der Schichtdicke der Lochtransportschicht gesteuert wird. Hier gelten bereits Verbesserungen im Bereich von 0.1 V als deutlicher Fortschritt.
4. Die Effizienz, Lebensdauer und Farbkoordinaten entsprechender Vorrichtungen sind vergleichbar zu Systemen gemäß dem Stand der Technik.
5. Die Prozessierbarkeit der erfindungsgemäßen Verbindungen ist deutlich besser im Vergleich zu Materialien gemäß dem Stand der Technik, welche eine ähnliche Struktur aufweisen, welche jedoch keine Substituenten R ungleich Wasserstoff oder Deuterium enthalten. So zeigen die erfindungsgemäßen Verbindungen keine Kristallisation am Rand der Aufdampfquelle und somit kein Verstopfen ("clogging") der Aufdampfquelle. Die erfindungsgemäßen Verbindungen sind daher besser für die Massenproduktion geeignet als Materialien gemäß dem Stand der Technik.

Im vorliegenden Anmeldetext und auch in den im Weiteren folgenden Beispielen wird auf die Verwendung der erfindungsgemäßen Verbindungen in Bezug auf OLEDs und die entsprechenden Displays und Beleuchtungselemente abgezielt. Trotz dieser Beschränkung der Beschreibung ist es für den Fachmann ohne weiteres erfinderisches Zutun möglich, die erfindungsgemäßen Verbindungen auch für weitere Verwendungen in anderen elektronischen Vorrichtungen einzusetzen, z. B. für organische Feld-Effekt-Transistoren (O-FETs), organische Dünnfilmtransistoren (O-TFTs), organische lichtemittierende Transistoren (O-LETs), organische integrierte Schaltungen (O-ICs), organische Solarzellen (O-SCs), organische Feld-Quench-Devices (O-FQDs), lichtemittierende elektrochemische Zellen (LECs), organische Photorezeptoren oder organische Laserdioden (O-Laser), um nur einige Anwendungen zu nennen.

Die Verwendung der erfindungsgemäßen Verbindungen in den entsprechenden Vorrichtungen ebenso wie diese Vorrichtungen selbst sind ebenfalls ein Gegenstand der vorliegenden Erfindung.

Die Erfindung wird durch die nachfolgenden Beispiele näher erläutert, ohne sie dadurch einschränken zu wollen.

### Beispiele:

Die nachfolgenden Synthesen werden, sofern nicht anders angegeben, unter einer Schutzgasatmosphäre in getrockneten Lösungsmitteln durchgeführt. Die Edukte können von ALDRICH bezogen werden.

### Beispiel 1: 4,6,6,10,12,12-Hexamethyl-N,N,N',N'-tetraphenyl-6H,12H-indeno[1,2-b]fluoren-2,8-diamin

### a) 2,2"-Dimethyl-[1,1';4',1"]terphenyl-2',5'-dicarbonsäurediethylester

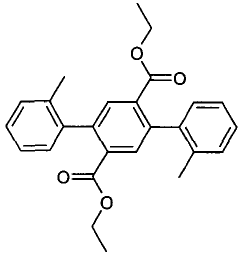

60 g (160 mmol) Dibromterephthalsäurediethylester, 43 g (320 mmol) o-Tolylboronsäure, 365 mg (0.32 mmol) Pd(PPh₃)₄ und 92 g (660 mmol) K₂CO₃ werden in 300 mL Toluol und 300 mL Wasser 4 h zum Sieden erhitzt. Das Gemisch wird im Anschluss zwischen Toluol und Wasser verteilt, die organische Phase dreimal mit Wasser gewaschen und über Na₂SO₄ getrocknet. Der verbleibende Rückstand wird zweimal aus Heptan zu farblosen Kristallen umkristallisiert. Die Ausbeute beträgt 51 g (127 mmol, 81 %).

### b) 2-[5'-(1-Hydroxy-1-methyl-ethyl)-2,2"-dimethyl-[1,1';4',1"]terphenyl-2'-yl]-propan-2-ol

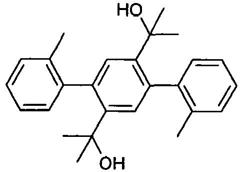

39 g (97 mmol) des Diesters werden in 500 mL getrocknetem THF vorgelegt, die Suspension bei 5 °C mit 195 mL (580 mmol) einer 3 M Methylmagnesiumchlorid-Lösung in THF versetzt und das Gemisch bei 5 °C für 6 h gerührt. Nach dieser Zeit erfolgt die Zugabe von 200 mL einer gesättigten NH₄Cl Lösung, das Reaktionsgemisch wird zwischen Wasser und Toluol verteilt, die wässrige Phase dreimal mit Toluol extrahiert und die vereinigten organischen Phasen über Na₂SO₄ getrocknet. Nach Entfernen des Lösungsmittels im Vakuum verbleiben 36 g (96 mmol, 99 %) eines farblosen Feststoffes, der DC- und ¹H-NMR einheitlich ist und ohne weitere Reinigung in die Folgereaktion eingesetzt werden kann.

### c) 4,6,6,10,12,12-Hexamethyl-6,12-dihydro-indeno[1,2-b]fluoren

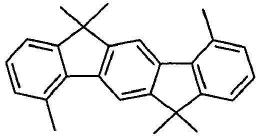

33.7 g (90 mmol) des Diols werden in 300 mL Dichlormethan gelöst, auf 5 °C abgekühlt und mit einer Mischung aus 80 g Polyphosphorsäure in 55 mL Methansulfonsäure versetzt. Nach 30 min bei 5 °C wurden 300 mL EtOH zugegeben und das Gemisch für 1 h zum Sieden erhitzt. Der farblose Niederschlag wird abfiltriert, zweimal mit EtOH und Heptan gewaschen und einmal aus Chlorbenzol umkristallisiert. Man erhält das Dimethylindenofluoren als farblosen Feststoff (28.5 g, 84 mmol, 93 %), der nach RP-HPLC eine Reinheit von > 99.8 % aufweist.

### d) 2,8-Dibrom-4,6,6,10,12,12-hexamethyl-6,12-dihydro-indeno[1,2-b]fluoren

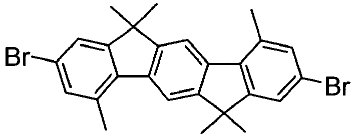

33.8 g (100 mmol) Dimethylindenofluoren werden in 1250 mL Dichlormethan gelöst, auf 0 °C gekühlt und mit einer Lösung von 33 g (266 mmol) Na₂CO₃ in 825 mL Wasser gemischt. Nach Zugabe von 11.3 mL Brom (220 mmol) wird die Suspension 6 h bei 5 °C gerührt, der farblose Feststoff abfiltriert, mit Wasser, EtOH und Heptan gewaschen und getrocknet. Es verbleiben 38.3 g (77 mmol, 77 %), Reinheit > 99.5 % (¹H-NMR).

### e) 4,6,6,10,12,12-Hexamethyl-N,N,N',N'-tetraphenyl-6H,12H-indeno[1,2-b]fluoren-2,8-diamin

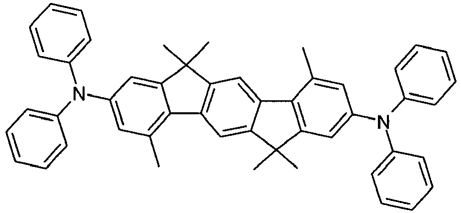

38 g (77 mmol) des Dibromids werden in 1400 mL trockenem Toluol gelöst, 31.1 g (183 mmol) Diphenylamin zugegeben und 30 min mit Ar gesättigt. Im Anschluss erfolgt die Zugabe von 23.5 g (245 mmol) NaO*^{t}*Bu, 3.9 mL (3.9 mmol) einer 1 M Lösung von Tri-*tert*-butylphosphin in Toluol, sowie 515 mg Pd(OAc)₂. Das Gemisch wird für 4 h zum Sieden erhitzt, zu der Suspension 500 mL Wasser, 50 mL konz. HCl und 500 mL EtOH gegeben, der Niederschlag abgesaugt, zweimal mit Wasser und MeOH gewaschen und getrocknet. Der farblose Feststoff wird in siedendem Dichlorbenzol gelöst, über eine Kieselgelschicht filtriert, mit siedendem Dichlorbenzol gewaschen, der im Filtrat ausgefallene Feststoff abgesaugt und dreimal aus NMP umkristallisiert. Nach zweifacher Sublimation im Vakuum (360 °C, 1 x 10⁻⁵ mbar) erhält man das Diamin (40.2 g, 60 mmol, 78 %) in Form eines blassgelben Pulvers, das eine durch RP-HPLC bestimmte Reinheit von > 99.9 % aufweist.

### Beispiel 2: 5,6,11,11,12,12-Hexamethyl-N,N,N',N'-tetraphenyl-11H,12H-indeno[2,1-a]fluoren-2,9-diamin

### a) 1,4-Dibrom-2,3-dimethyl-benzol

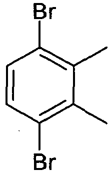

Die Verbindung wird in Anlehnung an Cachia, Wahl, Bull. Soc. Chim. Fr. 1958, 1418 -1420 hergestellt.

### b) 2,3-Dimethyl-benzol-1,4-bisboronsäurepinacolylester

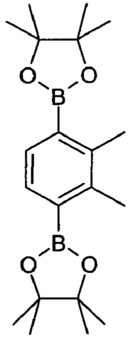

100 g (380 mmol) 1,4-Dibrom-2,3-dimethylbenzol werden in 1500 mL trockenem Diethylether gelöst, bei -70 °C 420 mL (840 mmol) einer 2 M Lösung von n-Butyllithium in Cyclohexan zugetropft, nach 1 h 130 mL Trimethylborat (1140 mmol) zugetropft, innerhalb 1 h auf RT kommen gelassen, das Lösungsmittel entfernt, 90 g (76 mmol) Pinacol sowie 1000 mL Toluol zugegeben, 2 h zum Sieden erhitzt, das Lösungsmittel erneut entfernt und der Rückstand, der nach ¹H-NMR einheitlich ist, ohne weitere Reinigung in die Folgereaktion eingesetzt.

### c) 2',3'-Dimethyl-[1,1';4',1"]terphenyl-2,2"-dicarbonsäuredimethylester

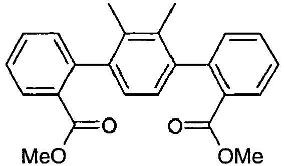

57 g (160 mmol) 2,3-Dimethylbenzol-1,4-bisboronsäurepinacolylester werden 4 h in einer Mischung aus 1150 mL EtOH, 1150 mL Toluol, 600 mL einer 2 M Na₂CO₃ Lösung, 45 mL (320 mmol) Methyl-2-brom-benzoat und 7.5 g (7 mmol) Pd(PPh₃)₄ zum Sieden erhitzt. Im Anschluss wird der Ansatz in eine Mischung aus Eiswasser/MeOH/HCl 1:1:1 gegossen, der farblose Niederschlag abgesaugt, mit Wasser und EtOH gewaschen und getrocknet. Der Feststoff wird in siedendem Toluol gelöst, über eine Kieselgelschicht filtriert, das Filtrat mit Heptan versetzt und das ausgefallene Produkt abgesaugt. Man erhält 55.4 g (148 mmol, 92 %) des Diesters als farbloses Pulver.

### d) 2-[2"-(1-Hydroxy-1-methyl-ethyl)-2',3'-dimethyl-[1,1';4',1"]terphenyl-2-yl]-propan-2-ol

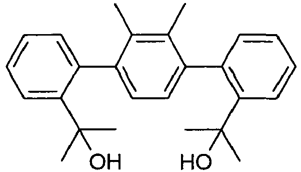

55,4 g (148 mmol) des Diesters werden in 850 mL THF gelöst, bei -75 °C mit 300 ml (600 mmol) einer 2 M Lösung von Methyllithium in Diethylether versetzt und 3 h bei -75 °C gerührt. Nach Erwärmen auf RT wird mit NH₄Cl-Lösung hydrolysiert, mit Essigsäurethylester extrahiert, getrocknet und im Vakuum vom Lösungsmittel befreit. Der verbleibende farblose Feststoff wird zweimal aus Toluol/EtOH umkristallisiert. Es verbleiben 42.7 g (114 mmol, 77 %) des Diols in Form farbloser Kristalle.

### e) 5,6,11,11,12,12-Hexamethyl-11,12-dihydro-indeno[2,1-a]fluoren

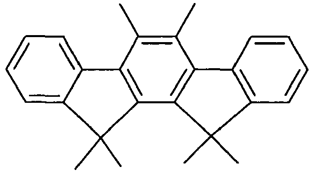

41.2 g (110 mmol) des Diols werden in 500 mL Essigsäure suspendiert, 1 mL konz. HCl zugegeben und das Gemisch für 4 h unter intensivem Rühren zum Sieden erhitzt. Nach Abkühlen auf RT wird der leicht gelbe Niederschlag abgesaugt, mit Wasser, MeOH und Heptan gewaschen und aus Chlorbenzol umkristallisiert. Man erhält 33.9 g (100 mmol, 91 %) *cis-*Indenofluoren mit einer Reinheit von > 99 % (RP-HPLC).

### f) 2,9-Dibrom-5,6,11,11,12,12-hexamethyl-11,12-dihydro-indeno[2,1-a]fluoren

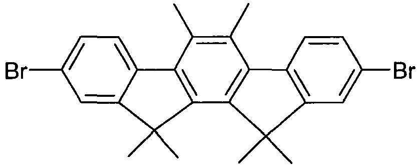

26.9 g (79.4 mmol) Dimethyl-*cis*-indenofluoren werden in 870 mL Dichlormethan gelöst, eine Lösung von 23.6 g (190 mmol) Na₂CO₃ Hydrat in 580 mL Wasser zugegeben und innerhalb 15 min bei 5 °C 9 mL (176 mmol) Brom in 40 mL Dichlormethan zugetropft. Die Suspension wird für 6 h bei 5 °C gerührt, der farblose Feststoff abfiltriert, mit Wasser und MeOH gewaschen und getrocknet. Man erhält 37.8 g (76.2 mmol, 96 %) des Dibromids als farbloses Pulver mit einer per ¹H-NMR bestimmten Reinheit von > 99 %.

### g) 5,6,11,11,12,12-Hexamethyl-N,N,N',N'-tetraphenyl-11H,12H-indeno[2,1-a]fluoren-2,9-diamin

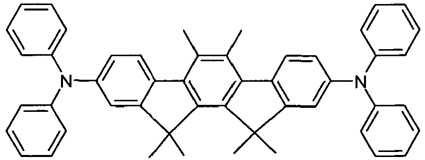

28.5 g (57.4 mmol) *cis*-Indenofluorendibromid werden in 1000 mL trockenem Toluol gelöst, die Lösung sorgfältig mit Ar gesättigt und 23.3 g (137.83 mmol) Diphenylamin zugegeben. Nach Zugabe von 17.7 g Natrium-*tert*-butylat, 3 mL einer 1 M Lösung von Tri-*tert*-butylphosphin in Toluol und 385 mg Pd(OAc)₂ wird das Gemisch für 3 h zum Sieden erhitzt, danach abgekühlt, mit 100 mL Wasser, 50 mL konz. HCl und 500 mL EtOH versetzt, der Niederschlag abgesaugt, in Toluol gelöst, über Kieselgel filtriert und die Lösung i.V. eingeengt. Nach sechsmaliger Umkristallisation aus Chlorbenzol wird der farblose Feststoff im Vakuum sublimiert (p = 1 x 10⁻⁵ mbar, T = 375 °C). Das glasartig angefallene, farblose Produkt (26.2 g, 68 %) weist eine Reinheit von > 99.9% auf, bestimmt durch RP-HPLC.

### Beispiel 3: 4,7,11,11,12,12-Hexamethyl-N,N,N',N',tetraphenyl-11H,12H-indeno[2,1-a]fluoren-2,9-diamin

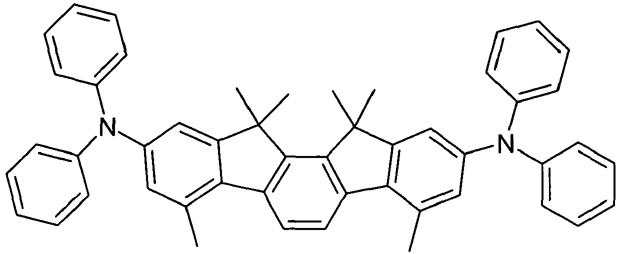

### a) O-Methylzimtaldehyd

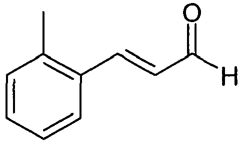

Die Verbindung kann gemäß Battistuzzi et al., Organic Letters 2003, 5, No. 5, 777-780 synthetisiert werden.

### b) Diethyl 2-Methylbenzylphosphonat

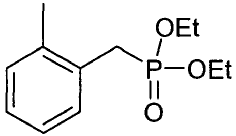

Die Verbindung kann gemäß de Meijere et al., Eur. J. Org. Chem. 1998, 2289-2299 synthetisiert werden.

### c) 2,2'-Dimethyl-trans-stilben

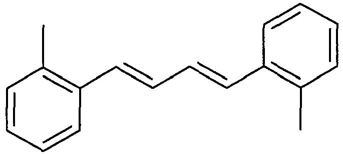

Die Verbindung kann gemäß de Meijere et al., Eur. J. Org. Chem. 1998, 2289-2299 synthetisiert werden.
5.99 g (149.9 mmol, 60%ige Suspension in Paraffinöl) NaH werden in 250 mL THF unter Argonatmosphäre suspendiert. Es wird auf 0 °C gekühlt und mit 37.28 g (153.9 mmol) Diethyl-2-methylbenzylphosphonat in 20 mL THF innerhalb von 15 min. versetzt. Es wird anschließend mit 19.74 g (135.0 mmol) o-Zimtaldehyd gelöst in 20 mL THF versetzt. Die Reaktion wurde anschließend bei RT für mehrere Tage gerührt. Man erhält 24.0 g (77%) des Produkts als weißen Feststoff.

### d) 1,2-Dicarboxymethyl-3,6-di-o-tolyl-1,2,4,5-cyclohexadien

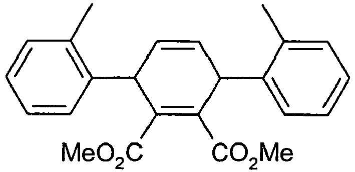

20.0 g (85.3 mmol) 2,2'-Dimethyl-trans-stilben werden mit 11.5 mL (93.8 mmol) Dimethylacetylendicarboxylat in Toluol 20 h unter Rückfluss gerührt. Man erhält einen Feststoff, der aus Heptan heiß ausgerührt wird. Das Produkt fällt als hellgelber Feststoff mit 87 % Ausbeute an.

### d) 1,2-Dicarboxymethyl-3,6-di-o-tolyl-benzol

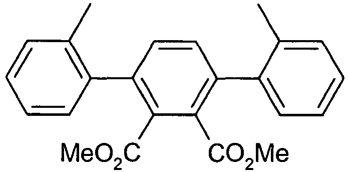

2.00 g (5.31 mmol) 1,2-Dicarboxymethyl-3,6-di-o-tolyl-1,2,4,5-cyclohexadien werden mit 9.23 g (10.62 mmol) MnO₂ in 250 mL o-Dichlorbenzol bei 180 °C am Wasserabscheider für 20 h gerührt. Man filtriert über Celite und erhält 1.3 g (65%) des Produkts als Feststoff.

### f) 1,2[1-Hydroxymethyl]-3,6-di-o-tolyl-benzol

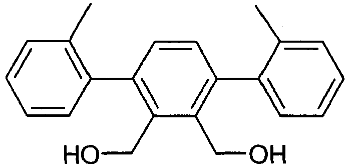

10.13 g (267.3 mmol) Lithiumaluminiumhydrid werden langsam mit 250 mL THF versetzt, dann langsam mit 71.5 g (190.9 mmol) 1,2-Dicarboxymethyl-3,6-di-o-tolyl-benzol, gelöst in 450 mL THF, versetzt, so dass die Temperatur nicht über 40 °C steigt. Es wird gegebenenfalls mit Trockeneis gekühlt. Nach vollständiger Zugabe wird die Suspension für 2 h unter Rückfluss erhitzt. Nach vollständigem Umsatz wird unter Kühlung bei 0 °C langsam mit einem Gemisch aus 120 mL Essigsäureethylester und 340 mL THF versetzt (Abreaktion des überschüssigen Lithiumaluminiumhydrids mit Essigsäureethylester). Nach vollständigem Quenchen wird vorsichtig mit 300 mL Ethanol versetzt und wässrig aufgearbeitet. Es wird mit 200 mL Wasser versetzt, der Niederschlag abgesaugt und die Mutterlauge etwas eingeengt, mit Essigsäureethylester und Dichlormethan extrahiert, über MgSO₄ getrocknet und eingeengt. Man erhält 61 g Rohprodukt, was direkt in die nächste Transformation eingesetzt werden kann.

### g) 4,7,Dimethyl-indeno[2,1-a]fluoren

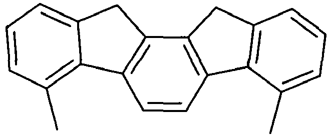

934 g Polyphosphorsäure werden vorgelegt, und es wird mit 61.0 g (192 mmol) 1,2[1-Hydroxymethyl]-3,6-di-o-tolyl-benzol, gelöst in 1500 mL Chloroform, versetzt. Das Gemisch wird anschließend für 2.5 h unter Rückfluss erhitzt. Die abgekühlte Reaktionslösung wird in Eiswasser eingetragen, mit Methylenchlorid extrahiert, getrocknet und eingeengt. Der erhaltene Feststoff wird in siedendem Essigester ausgerührt, abgesaugt und mit Heptan nachgewaschen. Man erhält 27 g (54 %) des Produkts als weißen Feststoff.

### h) 4,7,11,11,12,12,Hexamethyl-11,12-dihydro-indeno[2,1-a]fluoren

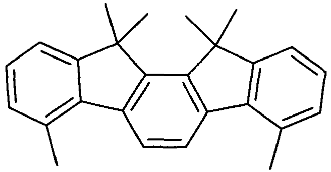

30.0 g (63.74 mmol) des 4,7,Dimethyl-indeno[2,1-a]fluoren werden in 700 mL DMSO vorgelegt und mit 36.76 g (382.47 mmol) NaO^{t}Bu versetzt. Das Gemisch wird auf 65 °C Innentemperatur erhitzt und dann mit 23.8 mL (382.47 mmol) lodmethan tropfenweise versetzt, so dass die Innentemperatur nicht über 65 °C steigt. Nach vollständigem Umsatz wird die abgekühlte Reaktionslösung mit 100 mL 26 %ige NH₃-Lösung versetzt und für 16 h bei Raumtemperatur gerührt. Das ausfallende Reaktionsprodukt wird abgesaugt und mit Toluol azeotrop getrocknet. Das Reaktionsprodukt wird via Säulenchromatographie (Toluol/Heptan 1:5) gereinigt. Man erhält 18 g (83 %) des Produkts als weißen Feststoff.

Die Bromierung und Hartwig-Buchwald-Kupplung dieser Verbindung erfolgt in Analogie zu Beispiel 1 f) und g).

### Beispiel 4: Herstellung der OLEDs

Die Herstellung von erfindungsgemäßen OLEDs erfolgt nach einem allgemeinen Verfahren gemäß WO 04/058911, das auf die hier beschriebenen Gegebenheiten (Schichtdickenvariation, verwendete Materialien) angepasst wird.

In den folgenden Beispielen 5 bis 18 werden die Ergebnisse verschiedener OLEDs vorgestellt. Glasplättchen, die mit strukturiertem ITO (Indium Zinn Oxid) beschichtet sind, bilden die Substrate der OLEDs. Zur verbesserten Prozessierung wird 20 nm PEDOT (aus Wasser aufgeschleudert; bezogen von H. C. Starck, Goslar, Deutschland; Poly(3,4-ethylendioxy-2,5-thiophen)) auf das Substrat aufgebracht. Die OLEDs bestehen aus folgender Schichtenfolge: Substrat / PEDOT 20 nm / HIL1 5 nm / Lochtransportschicht (HTM) 20 oder 110 nm / NPB 20 nm / Emissionschicht (EML) 30 nm / Elektronentransportschicht (ETM) 20 nm und abschließend eine Kathode. Die Materialien bis auf PEDOT werden in einer Vakuumkammer thermisch aufgedampft. Dabei besteht die Emissionsschicht immer aus einem Matrixmaterial (Host) und einem Dotierstoff (Dotand), der durch Coverdampfung dem Host beigemischt wird. Die Kathode wird durch eine 1 nm dicke LiF-Schicht und eine darauf abgeschiedene 100 nm dicke Al-Schicht gebildet. Tabelle 1 zeigt die chemischen Strukturen der verwendeten Materialien.

Diese OLEDs werden standardmäßig charakterisiert; hierfür werden die Elektrolumineszenzspektren, die Effizienz (gemessen in cd/A), die Leistungseffizienz (gemessen in Im/W) in Abhängigkeit der Helligkeit, berechnet aus Strom-Spannungs-Helligkeit-Kennlinien (IUL-Kennlinien), und die Lebensdauer bestimmt. Als Lebensdauer wird die Zeit definiert, nach der die Anfangshelligkeit von 25000 cd/m² auf die Hälfte gesunken ist. Die Einsatzspannung ist definiert als diejenige Spannung, bei der die OLED eine Helligkeit von 1 cd/m² erreicht.

In Tabelle 2 sind die Ergebnisse einiger OLEDs (Beispiele 5 bis 18) zusammengefasst. Als erfindungsgemäße Lochtransportmaterialien werden die Verbindungen HTM3 (Synthesebeispiel 1), HTM4 (Synthesebeispiel 2) und HTM5 (Synthesebeispiel 3) verwendet, wie in Tabelle 1 abgebildet. Als Vergleich werden die Verbindungen HTM1 und HTM2 gemäß dem nächstliegenden Stand der Technik verwendet. Dabei zeichnen sich die Verbindungen HTM3, HTM4 und HTM5 gegenüber den entsprechenden nicht substituierten Verbindungen HTM1 bzw. HTM2 durch eine verringerte Einsatzspannung, eine verringerte Betriebsspannung und vor allem eine verringerte Spannungsdifferenz zwischen 20 nm und 110 nm dicken HTM-Schichten bei einer Helligkeit von 1000 cd/m² aus. Dies ist in Anwendungen wichtig, da die optische Auskoppeleffizienz maßgeblich durch eine Variation der Schichtdicke der Lochtransportschicht gesteuert wird. Die Lebensdauern, Effizienzen und Farbkoordinaten beim Einsatz der erfindungsgemäßen Verbindungen HTM3, HTM4 und HTM5 sind erwartungsgemäß sehr ähnlich wie bei der Verwendung der entsprechenden Referenzmaterialien HTM1 und HTM2.

**Tabelle 1**

| | |
|---|---|
| 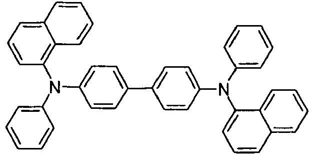 | 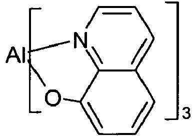 |
| NPB | AlQ₃ |
| 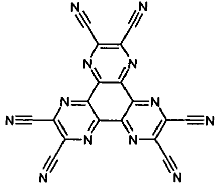 | 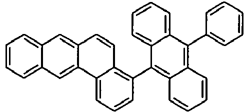 |
| HIL1 | H1 |
| 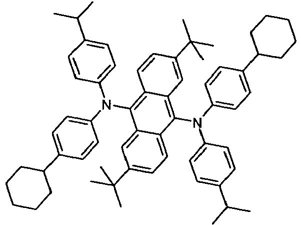 | 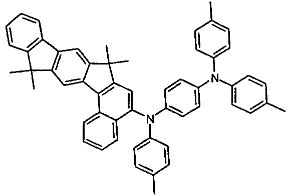 |
| D1 | D2 |
| 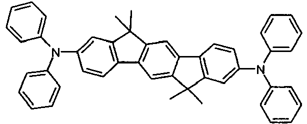 | 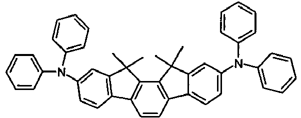 |
| HTM1 | HTM2 |
| 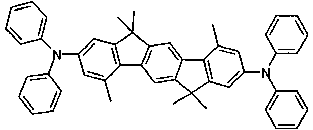 | 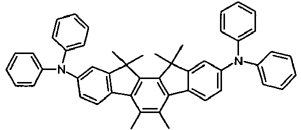 |
| HTM3 (Beispiel 1) | HTM4 (Beispiel 2) |
| 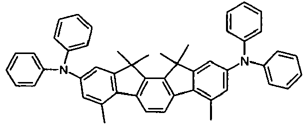 | |
| HTM5 (Beispiel 3) | |

**Tabelle 2**

| Bsp. | EML | HTM | Einsatzspannung | Spannung für 1000 cd/m² | Effizienz bei 1000 cd/m² | CIE x/y bei 1000 cd/m² | Lebensdauer [h] |
|---|---|---|---|---|---|---|---|
| 5 (Vgl.) | H1 + 10% D1 | HTM 1 20 nm | 2.8 V | 5.0 V | 17.1 cd/A | 0.34/0.62 | 355 |
| 6 (Vgl.) | H1 + 10% D1 | HTM 1 110 nm | 3.2 V | 5.5 V | 21.4 cd/A | 0.31/0.65 | 277 |
| 7 | H1 + 10% D1 | Bsp. HTM3 20 nm | 2.8 V | 4.9 V | 16.7 cd/A | 0.34/0.61 | 348 |
| 8 | H1 + 10% D1 | Bsp. HTM3 110 nm | 3.1 V | 5.2 V | 21.9 cd/A | 0.32/0.65 | 291 |
| 9 (Vgl.) | H1 + 10% D2 | HTM 1 20 nm | 2.8 V | 5.1 V | 14.4 cd/A | 0.32/0.58 | 361 |
| 10 (Vgl.) | H1 + 10% D2 | HTM 1 110 nm | 2.9 V | 5.4 V | 15.1 cd/A | 0.28/0.62 | 294 |
| 11 | H1 + 10% D2 | HTM3 20 nm | 2.8 V | 5.0 V | 14.9 cd/A | 0.32/0.58 | 365 |
| 12 | H1 + 10% D2 | HTM3 110 nm | 2.9 V | 5.2 V | 15.3 cd/A | 0.28/0.62 | 285 |
| 13 (Vgl.) | H1 + 10% D2 | HTM 2 20 nm | 4.2 V | 6.2 V | 15.8 cd/A | 0.31/0.58 | 285 |
| 14 (Vgl.) | H1 + 10% D2 | HTM 2 110 nm | 4.6 V | 6.5 V | 20.6 cd/A | 0.28/0.61 | 325 |
| 15 | H1 + 10% D2 | HTM4 20 nm | 3.5 V | 5.4 V | 15.2 cd/A | 0.31/0.58 | 315 |
| 16 | H1 + 10% D2 | HTM4 110 nm | 3.8 V | 5.6 V | 20.1 cd/A | 0.27/0.61 | 341 |
| 17 | H1 + 10% D2 | HTM5 20 nm | 2.9 V | 5.1 V | 16.2 cd/A | 0.32/0.59 | 338 |
| 18 | H1 + 10% D2 | HTM5 110 nm | 3.0 V | 5.2 V | 21.2 cd/A | 0.28/0.61 | 375 |

### Beispiel 19: Vergleich der Prozessierbarkeit

Für die Untersuchung der Prozessierbarkeit wurden HTM1 gemäß dem Stand der Technik und HTM3 als erfindungsgemäße Verbindung unter gleichen Aufdampfbedingungen mit einer Rate von 0.1 nm/s aufgedampft. Dabei wird die Aufdampfquelle jeweils nach 1 h und nach 2 h Bedampfung untersucht. Bereits nach 1 h Aufdampfung erkennt man eine deutliche Kristallisation von HTM1 am Rand der Aufdampfquelle, und nach 2 h Aufdampfung hat sich die Aufdampfquelle vollständig zugesetzt. Dagegen ist mit HTM3 auch nach 2 h Aufdampfung keinerlei Kristallisation am Rand der Aufdampfquelle und damit keinerlei Neigung zur Verstopfung der Aufdampfquelle ("clogging") zu erkennen. Dasselbe Ergebnis wird mit den erfindungsgemäßen Verbindungen HTM4 und HTM5 erhalten. Damit eignen sich die erfindungsgemäßen Verbindungen wesentlich besser zum Einsatz in der Massenproduktion als die Verbindung HTM1 gemäß dem Stand der Technik.

## Patentansprüche

1. Verbindungen gemäß Formel (1), 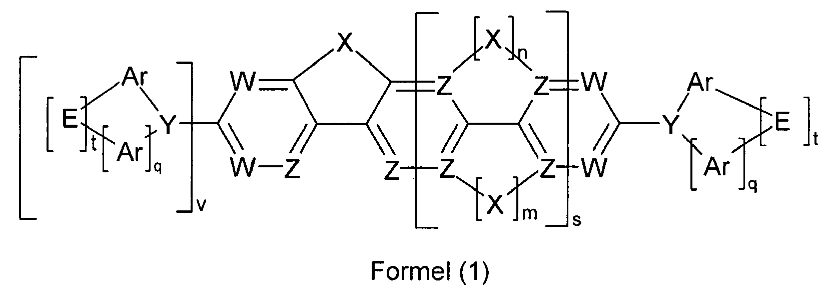 wobei für die verwendeten Symbole und Indizes gilt:
Y ist N;
Ar ist bei jedem Auftreten gleich oder verschieden ein aromatisches oder heteroaromatisches Ringsystem mit 5 bis 40 aromatischen Ringatomen, das mit einem oder mehreren Resten R¹ substituiert sein kann;
X ist bei jedem Auftreten gleich oder verschieden eine Gruppe, ausgewählt aus C(R¹)₂ und Si(R¹)₂;
Z ist C, wenn an die Gruppe Z eine Gruppe X gebunden ist, oder ist CR, wenn an die Gruppe Z keine Gruppe X gebunden ist;
W ist CH;
R ist bei jedem Auftreten gleich oder verschieden H, D, eine geradkettige Alkylgruppe mit 1 bis 10 C-Atomen oder eine verzweigte oder cyclische Alkylgruppe mit 3 bis 10 C-Atomen, die jeweils mit einem oder mehreren Resten R² substituiert sein können;
E ist bei jedem Auftreten gleich oder verschieden eine Einfachbindung, N(R¹), O, S, C(R¹)₂, C(R¹)₂-C(R¹)₂, Si(R¹)₂ oder B(R¹);
R¹ ist bei jedem Auftreten gleich oder verschieden H, D, F, Cl, Br, I, CN, NO₂, B(OR²)₂, Si(R²)₃, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch -R²C=CR²-, -C=C-, Si(R²)₂, Ge(R²)₂, Sn(R²)₂, C=O, C=S, C=Se, C=NR², -O-, -S-, -COO- oder -CONR²- ersetzt sein können und wobei ein oder mehrere H-Atome durch F, Cl, Br, I, CN oder NO₂ ersetzt sein können, oder ein aromatisches oder heteroaromatisches Ringsystem mit 5 bis 40 aromatischen Ringatomen, das durch einen oder mehrere nicht-aromatische Reste R¹ substituiert sein kann, oder eine Aryloxy- oder Hetero-aryloxygruppe mit 5 bis 40 aromatischen Ringatomen, die durch einen oder mehrere nicht-aromatische Reste R¹ substituiert sein kann, oder eine Kombination dieser Systeme; dabei können zwei oder mehrere Substituenten R¹ auch miteinander ein mono- oder polycyclisches Ringsystem bilden;
R² ist bei jedem Auftreten gleich oder verschieden H oder ein aliphatischer oder aromatischer Kohlenwasserstoffrest mit 1 bis 20 C-Atomen;
m, n sind 0 oder 1, mit der Maßgabe, dass m + n = 1 ist;
q ist bei jedem Auftreten 1;
s ist 1 oder 2;
t ist bei jedem Auftreten gleich oder verschieden 0 oder 1, wobei t = 0 bedeutet, dass statt der Gruppe E Reste R¹ gebunden sind; dabei gilt weiterhin, dass t = 0 ist, wenn q = 0 ist;
v ist 0 oder 1; dabei ist für v = 0 statt der Gruppe Y Wasserstoff oder ein Rest R gebunden;
**dadurch gekennzeichnet, dass** mindestens ein Rest R einen Substituenten ungleich H oder D darstellt.

2. Verbindungen nach Anspruch 1, ausgewählt aus den Verbindungen gemäß Formel (2) bis (3), 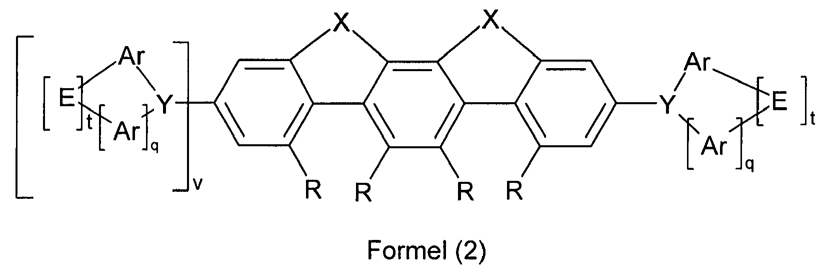 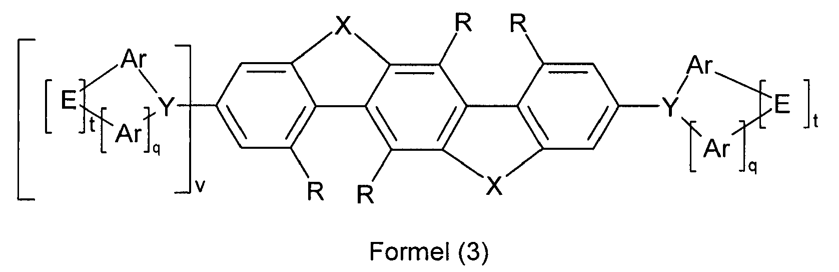 wobei die verwendeten Symbole und Indizes die in Anspruch 1 aufgeführten Bedeutungen haben und mindestens eine Gruppe R für einen Substituenten ungleich Wasserstoff oder Deuterium steht.

3. Verbindungen nach Anspruch 1 oder 2, **dadurch gekennzeichnet, dass** die Symbole Ar gleich oder verschieden bei jedem Auftreten für ein aromatisches oder heteroaromatisches Ringsystem mit 5 bis 16 aromatischen Ringatomen, für ein Triarylamin oder für Spirobifluoren stehen, das jeweils mit einem oder mehreren Resten R¹ substituiert sein kann, bevorzugt für ein aromatisches oder heteroaromatisches Ringsystem, ausgewählt aus Benzol, ortho-, meta- oder para-Biphenyl, Fluoren, Naphthalin, Anthracen, Phenanthren, Benzanthracen, Pyridin, Pyren, Thiophen, Triphenylamin, Diphenyl-1-naphthylamin, Diphenyl-2-naphthylamin, Phenyl-di(1-naphthyl)amin und Phenyl-di(2-naphthyl)amin, welches jeweils mit R¹ substituiert sein kann.

4. Verbindungen nach einem oder mehreren der Ansprüche 1 bis 3, **dadurch gekennzeichnet, dass** der Index t = 0 ist oder der Index t = 1 ist und das entsprechende Symbol E für eine Einfachbindung, C(R¹)₂, S oder N(R¹) steht.

5. Verbindungen nach einem oder mehreren der Ansprüche 1 bis 4, ausgewählt aus Verbindungen der Formeln (6) bis (9), 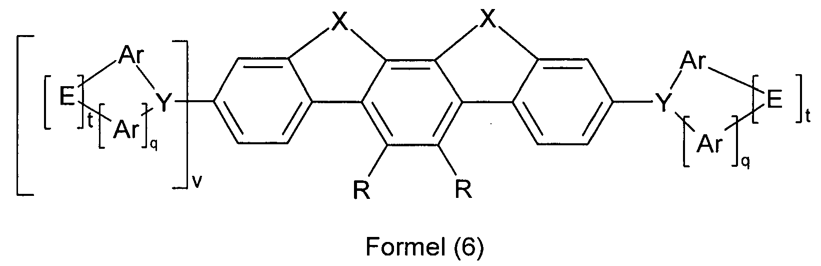 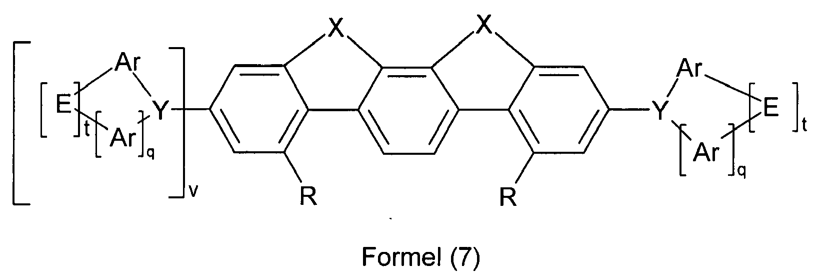 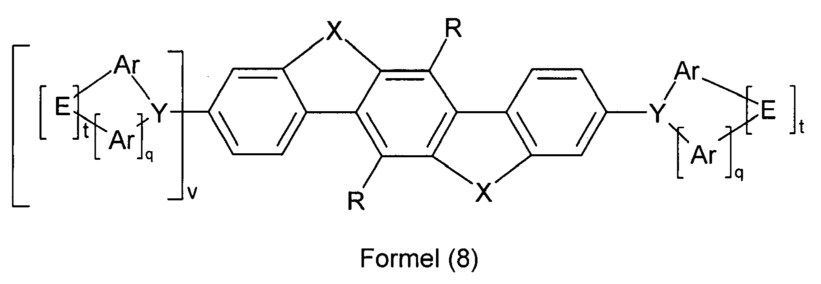 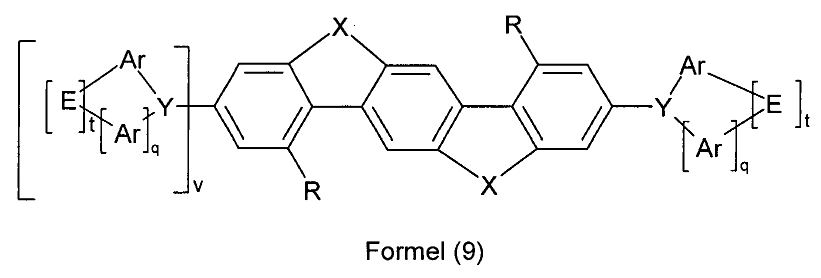 wobei die verwendeten Symbole und Indizes die in Anspruch 1 genannten Bedeutungen haben und die Reste R ungleich Wasserstoff oder Deuterium sind.

6. Verbindungen nach einem oder mehreren der Ansprüche 1 bis 5, **dadurch gekennzeichnet, dass** die Reste R ungleich Wasserstoff bei jedem Auftreten gleich oder verschieden Methyl, Ethyl, n-Propyl, i-Propyl, n-Butyl, i-Butyl, s-Butyl, t-Butyl, 2-Methylbutyl, n-Pentyl, s-Pentyl, tert-Penyl, 2-Pentyl, Cyclopentyl, n-Hexyl, s-Hexyl, tert-Hexyl, 2-Hexyl, 3-Hexyl, Cyclohexyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]octyl, 2-(2,6-Dimethyl)octyl oder 3-(3,7-Dimethyl)octyl sind.

7. Verbindungen nach einem oder mehreren der Ansprüche 1 bis 6, **dadurch gekennzeichnet, dass** die Symbole X gleichC(R¹)₂ sind.

8. Verbindungen nach einem oder mehreren der Ansprüche 1 bis 7, ausgewählt aus Verbindungen gemäß den Formeln (6a) bis (9a), 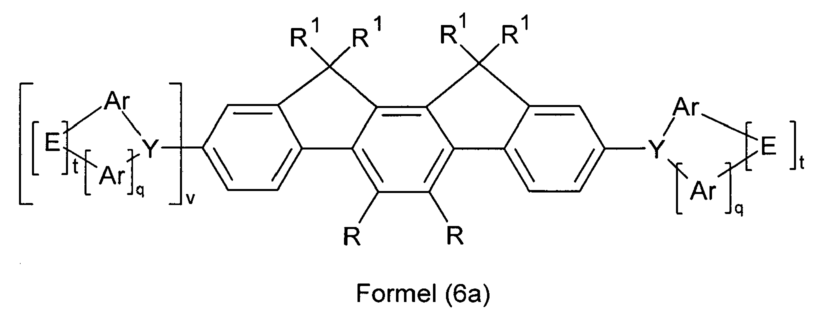 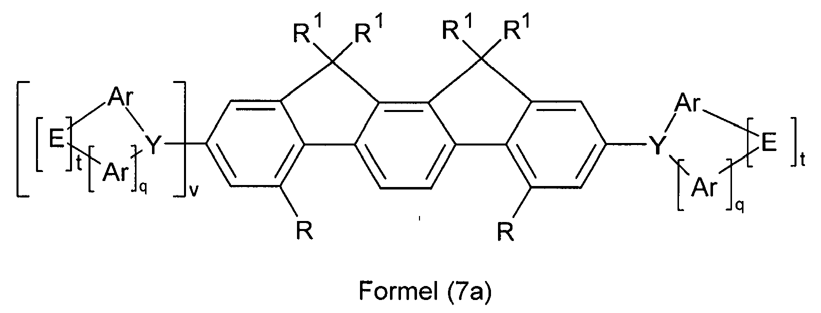 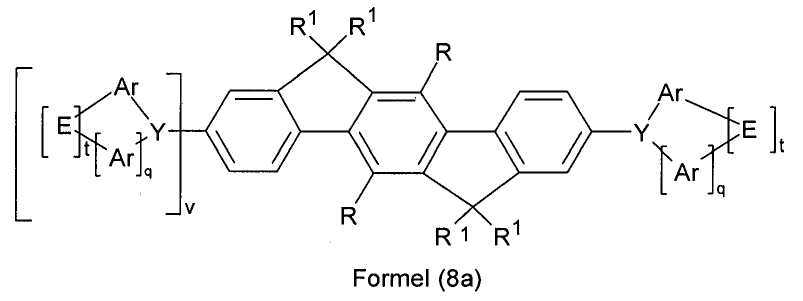 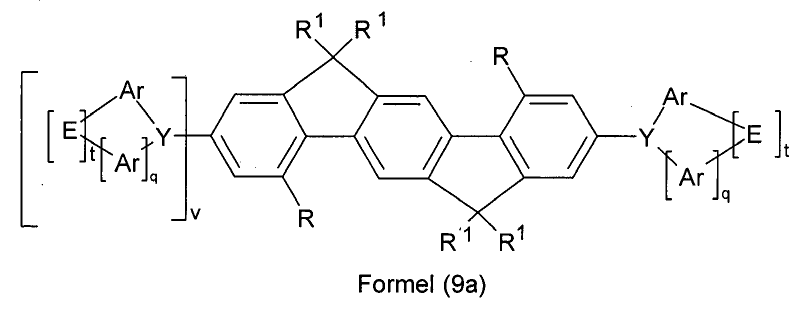 wobei die verwendeten Symbole und Indizes die in Anspruch 1 genannten Bedeutungen haben.

9. Verfahren zur Herstellung von Verbindungen nach einem oder mehreren der Ansprüche 1 bis 8 umfassend die Reaktionsschritte:
a) Synthese des Indenofluorengrundkörpers bzw. des entsprechenden heterocyclischen Derivats, welches statt der Gruppen Y in diesen Positionen Wasserstoff trägt;
b) Halogenierung, wobei ein Indenofluoren bzw. ein entsprechendes heterocyclisches Derivat entsteht, welches statt der Gruppen Y in diesen Positionen durch Halogen substituiert ist; und
c) Umsetzung des halogenierten Indenofluorens mit einem Diarylamin.

10. Verwendung von Verbindungen nach einem oder mehreren der Ansprüche 1 bis 8 in elektronischen Vorrichtungen, insbesondere in organischen Elektrolumineszenzvorrichtungen.

11. Organische elektronische Vorrichtungen, enthaltend mindestens eine Verbindung nach einem oder mehreren der Ansprüche 1 bis 8, insbesondere ausgewählt aus der Gruppe bestehend aus organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), lichtemittierenden elektrochemischen Zellen (LECs), organischen Photorezeptoren oder organischen Laserdioden (O-Laser).

12. Organische Elektrolumineszenzvorrichtung nach Anspruch 11, **dadurch gekennzeichnet, dass** die Verbindung gemäß einem oder mehreren der Ansprüche 1 bis 8 als Lochtransportmaterial und/oder als Lochinjektionsmaterial eingesetzt wird.

## Claims

1. Compounds of the formula (1) 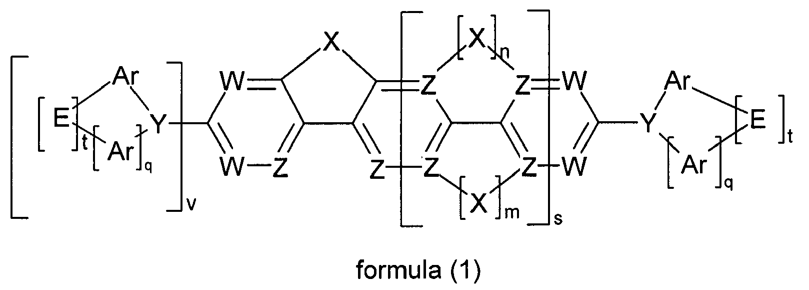 where the following applies to the symbols and indices used:
Y is N;
Ar is on each occurrence, identically or differently, an aromatic or heteroaromatic ring system having 5 to 40 aromatic ring atoms, which may be substituted by one or more radicals R¹;
X is on each occurrence, identically or differently, a group selected from C(R¹)₂ and Si(R¹)₂;
Z is C if a group X is bonded to the group Z or is CR if no group X is bonded to the group Z;
W is CH;
R is on each occurrence, identically or differently, H, D, a straight-chain alkyl group having 1 to 10 C atoms or a branched or cyclic alkyl group having 3 to 10 C atoms, each of which may be substituted by one or more radicals R²;
E is on each occurrence, identically or differently, a single bond, N(R¹), O, S, C(R¹)₂, C(R¹)₂-C(R¹)₂, Si(R¹)₂ or B(R¹);
R¹ is on each occurrence, identically or differently, H, D, F, Cl, Br, I, CN, NO₂, B(OR²)₂, Si(R²)₃, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy or thioalkoxy group having 3 to 40 C atoms, each of which may be substituted by one or more radicals R², where one or more non-adjacent CH₂ groups may be replaced by -R²C=CR²-, -C≡C-, Si(R²)₂, Ge(R²)₂, Sn(R²)₂, C=O, C=S, C=Se, C=NR², -0-, -S-, -COO- or -CONR²- and where one or more H atoms may be replaced by F, Cl, Br, I, CN or NO₂, or an aromatic or heteroaromatic ring system having 5 to 40 aromatic ring atoms, which may be substituted by one or more non-aromatic radicals R¹, or an aryloxy or heteroaryloxy group having 5 to 40 aromatic ring atoms, which may be substituted by one or more non-aromatic radicals R¹, or a combination of these systems; two or more substituents R¹ here may also form a mono- or polycyclic ring system with one another;
R² is on each occurrence, identically or differently, H or an aliphatic or aromatic hydrocarbon radical having 1 to 20 C atoms;
m, n are 0 or 1, with the proviso that m + n = 1;
q is on each occurrence 1;
s is 1 or 2;
t is on each occurrence, identically or differently, 0 or 1, where t = 0 means that radicals R¹ are bonded instead of the group E; furthermore, t = 0 if q = 0;
v is 0 or 1, where, for v = 0, hydrogen or a radical R is bonded instead of the group Y;
**characterised in that** at least one radical R represents a substituent other than H or D.

2. Compounds according to Claim 1, selected from the compounds of the formulae (2) to (3), 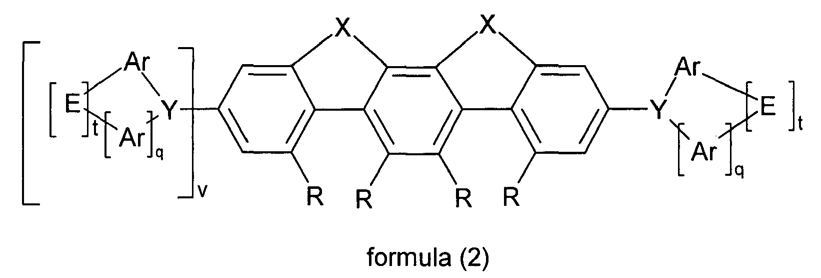 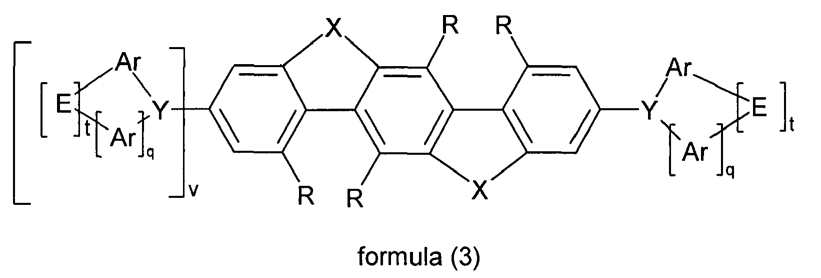 where the symbols and indices used have the meanings given in Claim 1 and at least one group R stands for a substituent other than hydrogen or deuterium.

3. Compounds according to Claim 1 or 2, **characterised in that** the symbols Ar stand, identically or differently on each occurrence, for an aromatic or heteroaromatic ring system having 5 to 16 aromatic ring atoms, for a triarylamine or for spirobifluorene, each of which may be substituted by one or more radicals R¹, preferably for an aromatic or heteroaromatic ring system selected from benzene, ortho-, meta- or para-biphenyl, fluorene, naphthalene, anthracene, phenanthrene, benzanthracene, pyridine, pyrene, thiophene, triphenylamine, diphenyl-1-naphthylamine, diphenyl-2-naphthylamine, phenyldi(1-naphthyl)amine and phenyldi(2-naphthyl)amine, each of which may be substituted by R¹.

4. Compounds according to one or more of Claims 1 to 3, **characterised in that** the index t = 0 or the index t = 1 and the corresponding symbol E stands for a single bond, C(R¹)₂, S or N(R¹).

5. Compounds according to one or more of Claims 1 to 4, selected from compounds of the formulae (6) to (9), 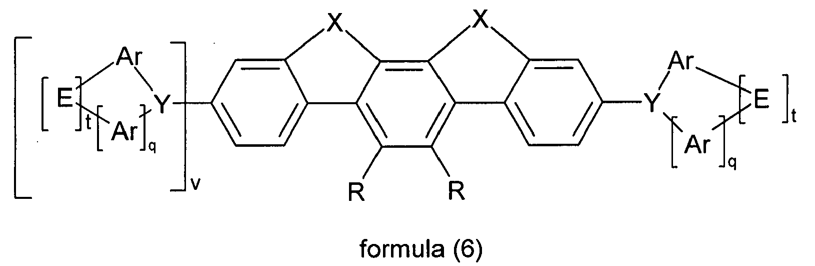 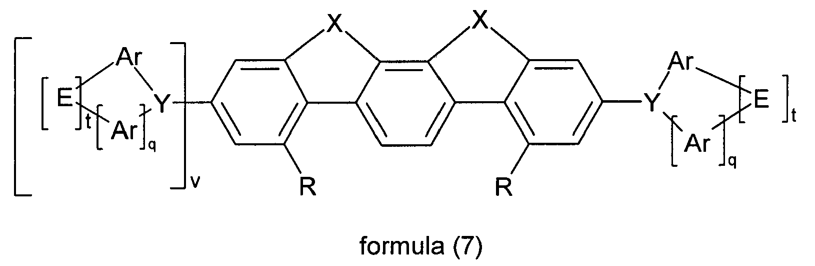 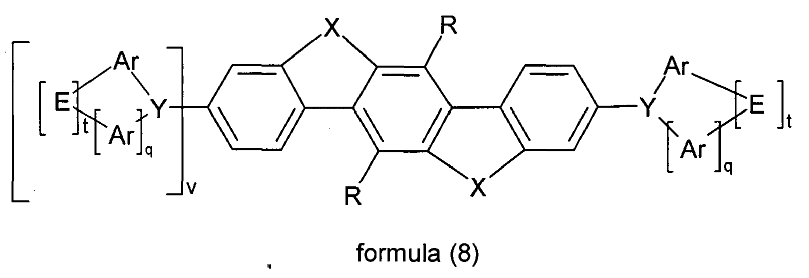 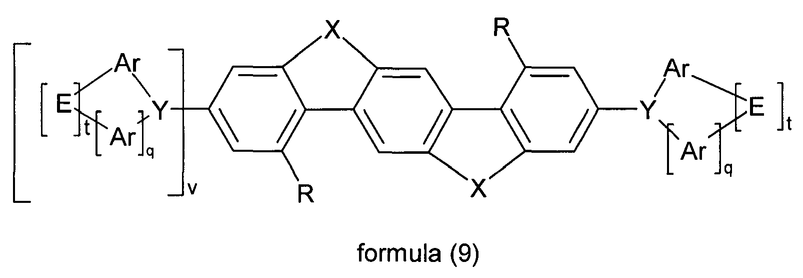 where the symbols and indices used have the meanings given in Claim 1 and the radicals R are other than hydrogen or deuterium.

6. Compounds according to one or more of Claims 1 to 5, **characterised in that** the radicals R other than hydrogen are on each occurrence, identically or differently, methyl, ethyl, n-propyl, i-propyl, n-butyl, i-butyl, s-butyl, t-butyl, 2-methylbutyl, n-pentyl, s-pentyl, tert-pentyl, 2-pentyl, cyclopentyl, n-hexyl, s-hexyl, tert-hexyl, 2-hexyl, 3-hexyl, cyclohexyl, 2-methylpentyl, n-heptyl, 2-heptyl, 3-heptyl, 4-heptyl, cycloheptyl, 1-methylcyclohexyl, n-octyl, 2-ethylhexyl, cyclooctyl, 1-bicyclo[2.2.2]octyl, 2-bicyclo[2.2.2]octyl, 2-(2,6-dimethyl)octyl or 3-(3,7-dimethyl)octyl.

7. Compounds according to one or more of Claims 1 to 6, **characterised in that** the symbols X are equal to C(R¹)₂.

8. Compounds according to one or more of Claims 1 to 7, selected from compounds of the formulae (6a) to (9a), 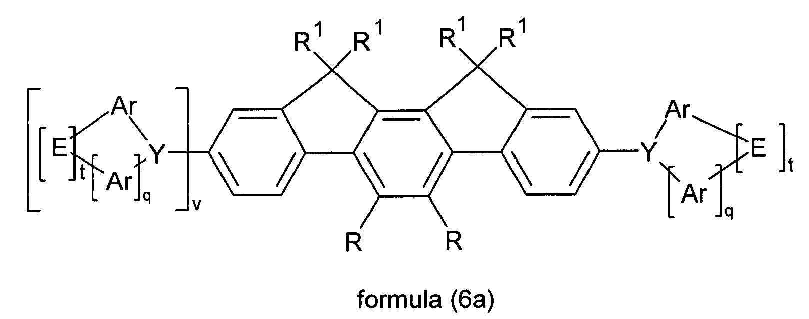 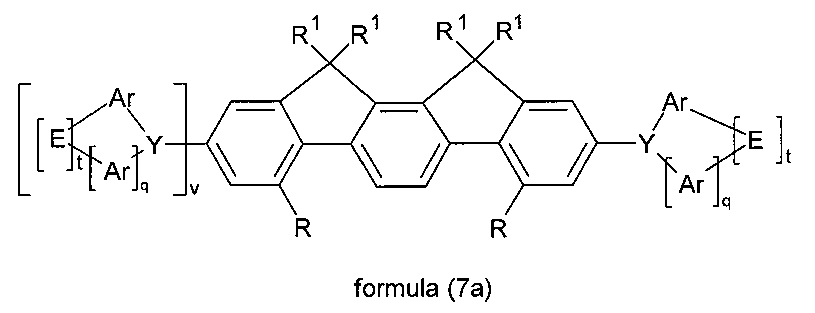 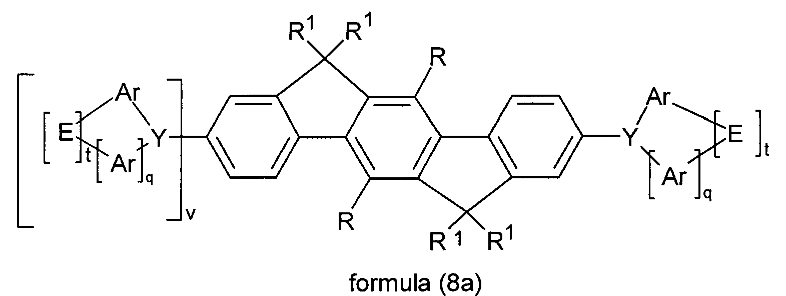 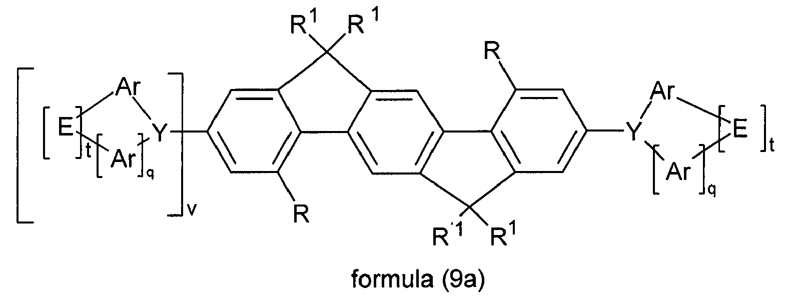 where the symbols and indices used have the meanings given in Claim 1.

9. Process for the preparation of compounds according to one or more of Claims 1 to 8, comprising the reaction steps:
a) synthesis of the indenofluorene skeleton or the corresponding heterocyclic derivative which carries hydrogen instead of the groups Y in these positions;
b) halogenation, giving an indenofluorene or a corresponding heterocyclic derivative which is substituted in these positions by halogen instead of the groups Y; and
c) reaction of the halogenated indenofluorene with a diarylamine.

10. Use of compounds according to one or more of Claims 1 to 8 in electronic devices, in particular in organic electroluminescent devices.

11. Organic electronic devices comprising at least one compound according to one or more of Claims 1 to 8, in particular selected from the group consisting of organic field-effect transistors (O-FETs), organic thin-film transistors (O-TFTs), organic light-emitting transistors (O-LETs), organic integrated circuits (O-ICs), organic solar cells (O-SCs), organic field-quench devices (O-FQDs), light-emitting electrochemical cells (LECs), organic photoreceptors and organic laser diodes (O-lasers).

12. Organic electroluminescent device according to Claim 11, **characterised in that** the compound according to one or more of Claims 1 to 8 is employed as hole-transport material and/or as hole-injection material.

## Revendications

1. Composés de la formule (1) : 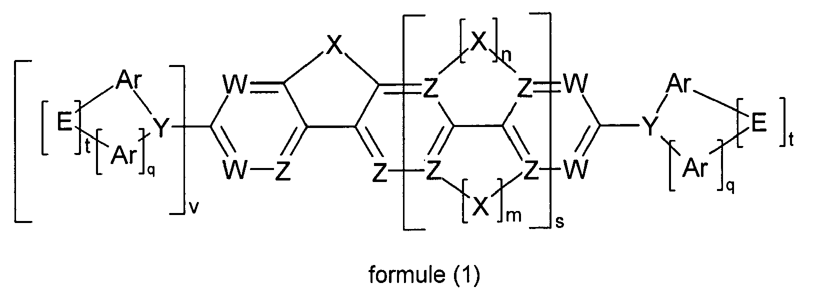 dans laquelle ce qui suit s'applique aux symboles et indices utilisés :
Y est N ;
Ar est, pour chaque occurrence, de manière identique ou différente, un système de cycle aromatique ou hétéroaromatique comportant 5 à 40 atomes de cycle aromatique, lequel peut être substitué par un radical ou plusieurs radicaux R¹ ;
X est, pour chaque occurrence, de manière identique ou différente, un groupe choisi parmi C(R¹)₂ et Si(R¹)₂ ;
Z est C si un groupe X est lié au groupe Z ou est CR si aucun groupe X n'est lié au groupe Z ;
W est CH ;
R est, pour chaque occurrence, de manière identique ou différente, H, D, un groupe alkyle en chaîne droite comportant 1 à 10 atome(s) de C ou un groupe alkyle ramifié ou cyclique comportant 3 à 10 atomes de C, dont chacun peut être substitué par un radical ou plusieurs radicaux R² ;
E est, pour chaque occurrence, de manière identique ou différente, une liaison simple, N(R¹), O, S, C(R¹)₂, C(R¹)₂-C(R¹)₂, Si(R¹)₂ ou B(R¹) ;
R¹ est, pour chaque occurrence, de manière identique ou différente, H, D, F, Cl, Br, I, CN, NO₂, B(OR²)₂, Si(R²)₃, un groupe alkyle, alcoxy ou thioalcoxy en chaîne droite comportant 1 à 40 atome(s) de C ou un groupe alkényle ou alkynyle en chaîne droite comportant 2 à 40 atomes de C ou un groupe alkyle, alkényle, alkynyle, alcoxy ou thioalcoxy ramifié ou cyclique comportant 3 à 40 atomes de C, dont chacun peut être substitué par un radical ou plusieurs radicaux R², où un ou plusieurs groupe(s) CH₂ non adjacent(s) peut/peuvent être remplacé(s) par -R²C=CR²-, -C≡C-, Si(R²)₂, Ge(R²)₂, Sn(R²)₂, C=O, C=S, C=Se, C=NR², -O-, -S-, -COO- ou -CONR²- et où un ou plusieurs atome(s) de H peut/peuvent être remplacé(s) par F, Cl, Br, I, CN ou NO₂, ou un système de cycle aromatique ou hétéroaromatique comportant 5 à 40 atomes de cycle aromatique, lequel peut être substitué par un radical ou plusieurs radicaux R¹ non aromatique(s), ou un groupe aryloxy ou hétéroaryloxy comportant 5 à 40 atomes de cycle aromatique, lequel peut être substitué par un radical ou plusieurs radicaux R¹ non aromatique(s), ou une combinaison de ces systèmes ; deux substituants R¹ ou plus peuvent ici également former un système de cycle monocyclique ou polycyclique l'un avec l'autre ou les uns avec les autres ;
R² est, pour chaque occurrence, de manière identique ou différente, H ou un radical hydrocarbone aliphatique ou aromatique comportant 1 à 20 atome(s) de C ;
m, n sont 0 ou 1, étant entendu que m + n = 1 ;
q est pour chaque occurrence 1 ;
s est 1 ou 2 ;
t est, pour chaque occurrence, de manière identique ou différente, 0 ou 1, où t = 0 signifie que des radicaux R¹ sont liés en lieu et place du groupe E ; qui plus est, t = 0 si q = 0 ;
v est 0 ou 1, où, pour v = 0, hydrogène ou un radical R est lié en lieu et place du groupe Y ;
**caractérisés en ce qu'**au moins un radical R représente un substituant autre que H ou que D.

2. Composés selon la revendication 1, choisis parmi les composés des formules (2) et (3) : 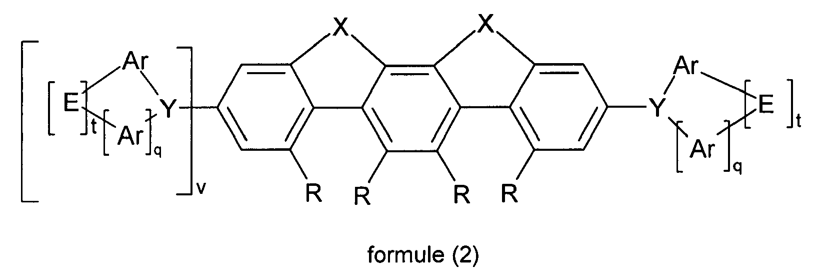 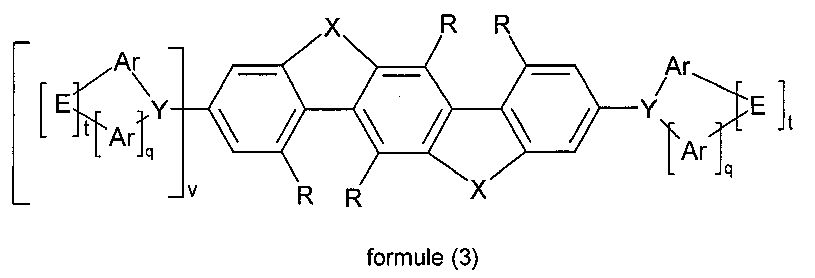 dans lesquelles les symboles et indices utilisés présentent les significations données selon la revendication 1 et au moins un groupe R représente un substituant autre qu'hydrogène ou que deutérium.

3. Composés selon la revendication 1 ou 2, **caractérisés en ce que** les symboles Ar représentent, de manière identique ou différente pour chaque occurrence, un système de cycle aromatique ou hétéroaromatique comportant 5 à 16 atomes de cycle aromatique, un triarylamine ou un spirobifluorène, dont chacun peut être substitué par un radical ou plusieurs radicaux R¹, de façon préférable un système de cycle aromatique ou hétéroaromatique choisi parmi benzène, ortho-, méta- ou para-biphényle, fluorène, naphtalène, anthracène, phénanthrène, benzanthracène, pyridine, pyrène, thiophène, triphénylamine, diphényl-1-naphtylamine, diphényl-2-naphtylamine, phényldi(1-naphtyl)amine et phényldi(2-naphtyl)amine, dont chacun peut être substitué par R¹.

4. Composés selon une ou plusieurs des revendications 1 à 3, **caractérisés en ce que** l'indice t = 0 ou l'indice t = 1 et le symbole correspondant E représente une liaison simple, C(R¹)₂, S ou N(R¹).

5. Composés selon une ou plusieurs des revendications 1 à 4, choisis parmi des composés des formules (6) à (9) : 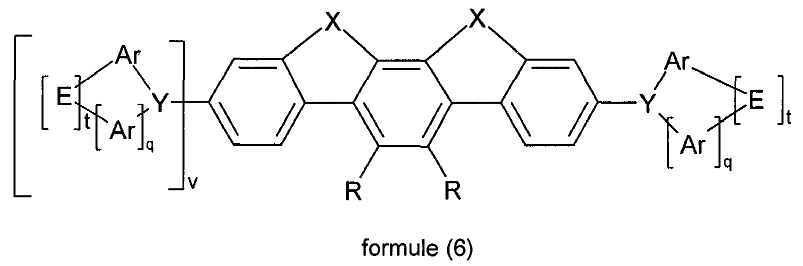 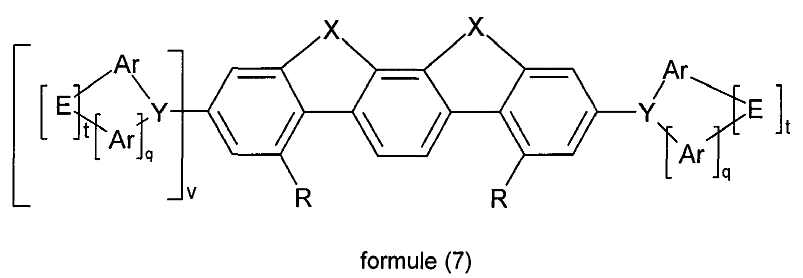 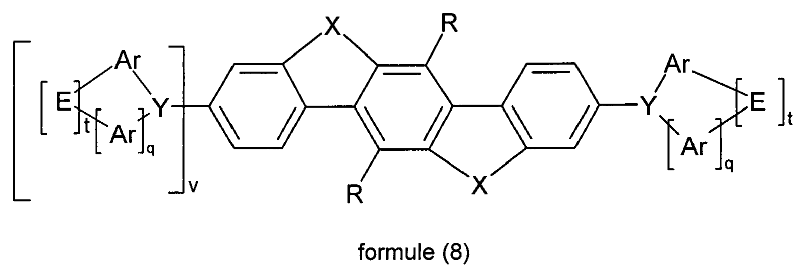 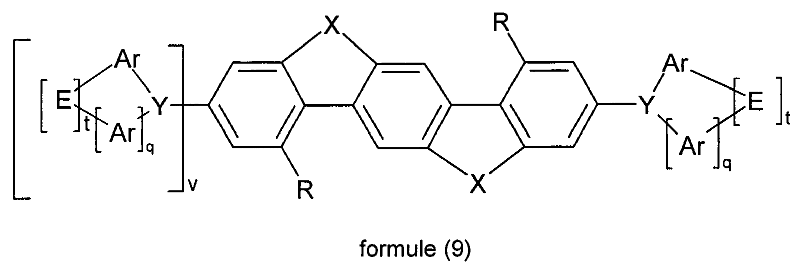 dans lesquelles les symboles et indices utilisés présentent les significations données selon la revendication 1 et les radicaux R sont autres qu'hydrogène ou que deutérium.

6. Composés selon une ou plusieurs des revendications 1 à 5, **caractérisés en ce que** les radicaux R autres qu'hydrogène sont, pour chaque occurrence, de manière identique ou différente, méthyle, éthyle, n-propyle, i-propyle, n-butyle, i-butyle, s-butyle, t-butyle, 2-méthyl-butyle, n-pentyle, s-pentyle, tert-pentyle, 2-pentyle, cyclopentyle, n-hexyle, s-hexyle, tert-hexyle, 2-hexyle, 3-hexyle, cyclohexyle, 2-méthylpentyle, n-heptyle, 2-heptyle, 3-heptyle, 4-heptyle, cyclo-heptyle, 1-méthylcyclohexyle, n-octyle, 2-éthylhexyle, cyclooctyle, 1-bicyclo[2.2.2]octyle, 2-bicyclo[2.2.2]octyle, 2-(2,6-diméthyl)octyle ou 3-(3,7-diméthyl)octyle.

7. Composés selon une ou plusieurs des revendications 1 à 6, **caractérisés en ce que** les symboles X sont égaux à C(R¹)₂.

8. Composés selon une ou plusieurs des revendications 1 à 7, choisis parmi des composés des formules (6a) à (9a) : 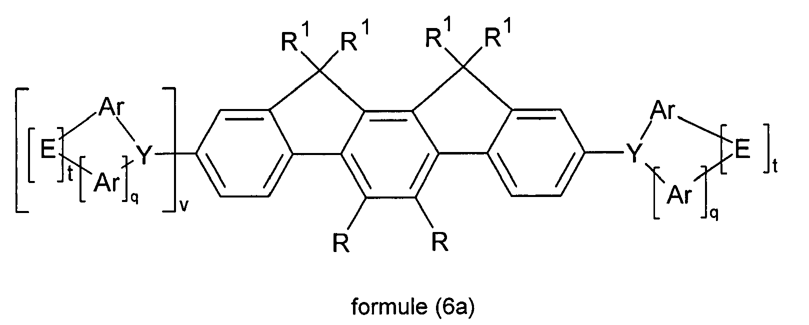 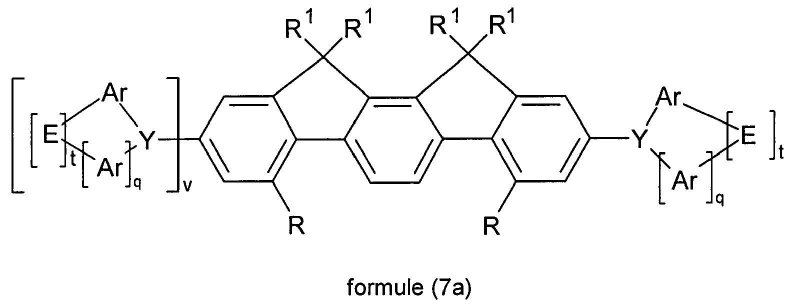 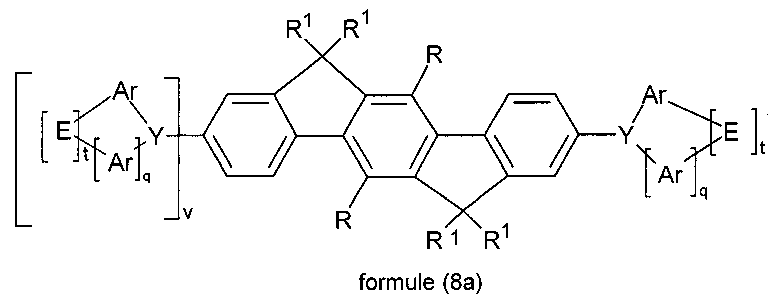 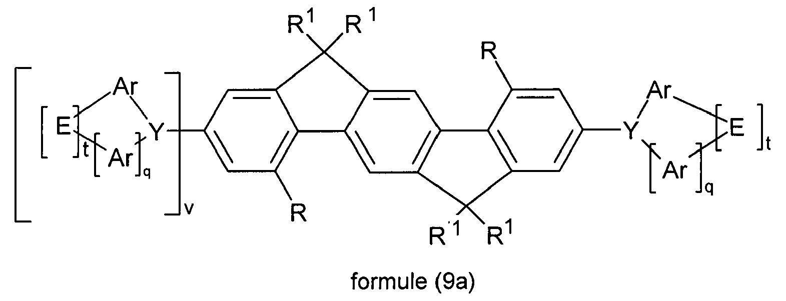
dans lesquelles les symboles et indices utilisés présentent les significations données selon la revendication1.

9. Procédé pour la préparation de composés selon une ou plusieurs des revendications 1 à 8, comprenant les étapes de réaction qui suivent :
a) synthèse du squelette indénofluorène ou du dérivé hétérocyclique correspondant, lequel est porteur d'hydrogène en lieu et place des groupes Y au niveau de ces positions ;
b) halogénation, d'où l'obtention d'un indénofluorène ou d'un dérivé hétérocyclique correspondant, lequel est substitué au niveau de ces positions par halogène en lieu et place des groupes Y ; et
c) réaction de l'indénofluorène halogéné avec un diarylamine.

10. Utilisation de composés selon une ou plusieurs des revendications 1 à 8 dans des dispositifs électroniques, en particulier dans des dispositifs électroluminescents organiques.

11. Dispositifs électroniques organiques comprenant au moins un composé selon une ou plusieurs des revendications 1 à 8, en particulier choisis parmi le groupe constitué par des transistors à effet de champ organiques (O-FET), des transistors à film mince organiques (O-TFT), des transistors à émission de lumière organiques (O-LET), des circuits intégrés organiques (O-IC), des cellules solaires organiques (O-SC), des dispositifs à extinction de champ organiques (O-FQD), des cellules électrochimiques à émission de lumière (LEC), des photorécepteurs organiques et des diodes laser organiques (O-laser).

12. Dispositif électroluminescent organique selon la revendication 11, **caractérisé en ce que** le composé selon une ou plusieurs des revendications 1 à 8 est utilisé en tant que matériau de transport de trous et/ou en tant que matériau d'injection de trous.
